(19) Europäisches Patentamt
European Patent Office
Office européen des brevets

(11) Veröffentlichungsnummer: **0 400 519 A1**

(12) # EUROPÄISCHE PATENTANMELDUNG

(21) Anmeldenummer: **90110052.9**

(22) Anmeldetag: **28.05.90**

(51) Int. Cl.5: **C07D 237/04, C07D 403/04, C07D 413/04, A61K 31/50**

(30) Priorität: **01.06.89 DE 3917801**
**14.10.89 DE 3934436**

(43) Veröffentlichungstag der Anmeldung:
**05.12.90 Patentblatt 90/49**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Hauel, Norbert, Dr. Dipl.-Chem.**
**Stresemannstrasse 40**
**D-7950 Biberach 1(DE)**
Erfinder: **Mauz, Annerose, Dr. Dipl.-Biol.**
**Habsbergerstrasse 59**
**D-7945 Langenenslingen-Emerfeld(DE)**
Erfinder: **van Meel, Jacques, Dr.**
**Schubertweg 4**
**D-7951 Mittelbiberach(DE)**
Erfinder: **Entzeroth, Michael, Dr. Dipl.-Chem.**
**Sebastian-Sailer-Strasse 20**
**D-7951 Warthausen(DE)**
Erfinder: **Zimmermann, Rainer, Dr. Dipl.-Biochem.**
**Laurenbühlstrasse 17**
**D-7951 Mittelbiberach(DE)**
Erfinder: **Diederen, Willi, Dr.**
**Haldenstrasse 1a**
**D-7950 Biberach 1(DE)**

(54) **2-Hydroxy-n-propylamine, diese Verbindungen enthaltende Arzneimittel und Verfahren zu ihrer Herstellung.**

(57) Die Erfindung betrifft neue 2-Hydroxy-n-propylamine der Formel
$R_1$-O-$CH_2$-CHOH-$CH_2$-NH-A-$R_2$ ,(I)
in der
$R_1$,$R_2$ und A wie im Anspruch 1 definiert sind, deren Tautomere, deren Enantiomere und deren Säureadditionssalze, insbesondere für die pharmazeutische Anwendung deren physiologisch verträglichen Säureadditionssalze mit anorganischen oder organischen Säuren, welche wertvolle pharmakologische Eigenschaften aufweisen, insbesondere bei einer gleichzeitigen cardiotonen eine $\beta$-blockierende Wirkung, und deren Verwendung als Arzneimittel sowie Verfahren zu ihrer Herstellung.

EP 0 400 519 A1

## 2-Hydroxy-n-propylamine, diese Verbindungen enthaltende Arzneimittel und Verfahren zu ihrer Herstellung

Gegenstand der vorliegenden Erfindung sind 2-Hydroxy-n-propylamine der Formel

$R_1$-O-$CH_2$-CHOH-$CH_2$-NH-A-$R_2$ ,(I)

deren Tautomere, deren Enantiomere und deren Säureadditionssalze, insbesondere für die pharmazeutische Anwendung deren physiologisch verträglichen Säureadditionssalze mit anorganischen oder organischen Säuren, deren Verwendung und Verfahren zu ihrer Herstellung.

Die obigen Verbindungen weisen wertvolle pharmakologische Eigenschaften auf, insbesondere eine gleichzeitige cardiotone und ß-blockierende Wirkung.

In der obigen Formel I bedeutet

$R_1$ eine Naphthylgruppe, eine über den Phenylring gebundene 3,4-Dihydro-carbostyrilgruppe, eine durch eine Alkylsulfonyloxygruppe mit 1 bis 5 Kohlenstoffatomen, durch eine Allyl-, Allyloxy-, Cyano- oder Aminocarbonylmethylgruppe substituierte Phenylgruppe, eine durch eine Alkylgruppe mit 1 bis 3 Kohlenstoffatomen substituierte Phenylgruppe, in welcher der Alkylteil in 1-, 2- oder 3-Stellung durch eine Alkoxy-, Cycloalkoxy-, Cycloalkylmethoxy-, 2-Alkoxyethoxy-, 2-Cycloalkoxyethoxy-, 2-Cycloalkylmethoxyethoxy- oder 2-Phenoxyethoxygruppe substituiert ist, wobei der Alkoxyteil jeweils 1 bis 6 Kohlenstoffatome und der Cycloalkyl- oder Cycloalkoxyteil jeweils 3 bis 6 Kohlenstoffatome enthalten kann, oder eine durch eine Alkylgruppe mit 1 bis 3 Kohlenstoffatomen und durch ein Halogenatom disubstituierte Phenylgruppe,

A eine gegebenenfalls durch eine oder zwei Alkylgruppen mit jeweils 1 bis 3 Kohlenstoffatomen substituierte geradkettige Alkylengruppe mit 2 bis 4 Kohlenstoffatomen und

$R_2$ eine Gruppe der Formel

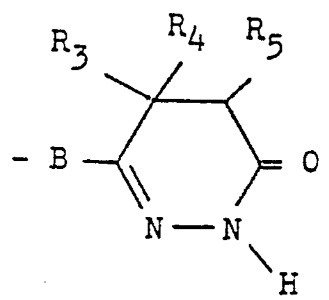

,

in welcher

$R_3$ ein Wasserstoffatom oder eine Alkylgruppe mit 1 bis 3 Kohlenstoffatomen,

$R_4$ und $R_5$ jeweils ein Wasserstoffatom oder zusammen eine weitere Kohlenstoff-Kohlenstoffbindung und

B eine Gruppe der Formeln

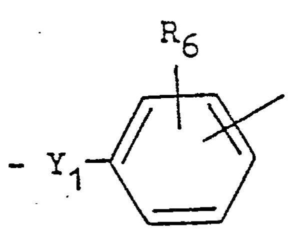

,

oder

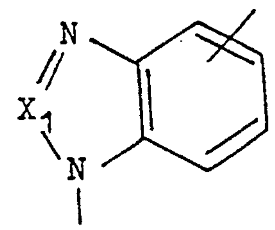

oder

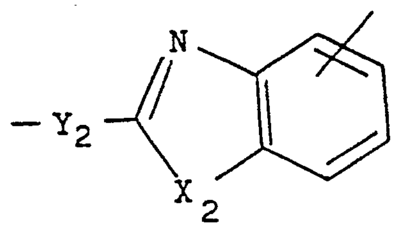

darstellen, in welchen
$R_6$ eine Alkylgruppe mit 1 bis 3 Kohlenstoffatomen, ein Wasserstoff- oder Halogenatom,
$Y_1$ eine Bindung, ein Sauerstoffatom, eine Sulfonylgruppe oder eine -NH-CO-$CH_2$-Gruppe, wobei die -$CH_2$-Gruppe mit dem Phenylkern verbunden ist,
$X_1$ ein Stickstoffatom oder eine gegebenenfalls durch eine Hydroxygruppe oder eine Alkylgruppe mit 1 bis 3 Kohlenstoffatomen substituierte Methingruppe,
$X_2$ ein Sauerstoffatom oder eine Iminogruppe und
$Y_2$ eine Bindung oder eine Iminogruppe bedeuten.

Für die bei der Definition der Reste eingangs erwähnten Bedeutungen kommt beispielsweise für $R_1$ die der 1-Naphthyl-, 2-Naphthyl-, 3,4-Dihydro-carbostyril-(5)-, 3,4-Dihydro-carbostyril-(6)-, 3,4-Dihydrocarbostyril-(7)-, 3,4-Dihydro-carbostyril-(8)-, 4-Methylsulfonyloxy-phenyl-, 4-Ethylsulfonyloxy-phenyl-, 4-n-Propylsulfonyloxy-phenyl-, 4-Isopropylsulfonyloxy-phenyl-, 4-n-Butylsulfonyloxy-phenyl-, 4-n-Pentylsulfonyloxy-phenyl-, 4-Isobutylsulfonyloxy-phenyl-, 4-tert.Pentylsulfonyloxy-phenyl-, 2-Allyl-phenyl-, 4-Allyl-phenyl-, 2-Allyloxy-phenyl-, 4-Allyloxy-phenyl-, 2-Cyanophenyl-, 3-Cyano-phenyl-, 4-Cyanophenyl-, 2-Aminocarbonylmethyl-phenyl-, 3-Aminocarbonylmethyl-phenyl-, 4-Aminocarbonylmethyl-phenyl-, 4-(Methoxymethyl)phenyl-, 4-(Ethoxymethyl)phenyl-, 4-(n-Propoxymethyl)phenyl-, 4-(Isopropoxymethyl)-phenyl-, 4-(Cyclopropoxymethyl)phenyl-, 4-(n-Butoxymethyl)phenyl-, 4-((2-Methyl-n-propoxy)methyl)phenyl-, 4-(n-Pentoxymethyl)phenyl-, 4-(n-Hexoxymethyl)phenyl-, 4-(Cyclopropylmethoxy-methyl)phenyl-, 4-(Cyclobutoxymethyl)phenyl-, 4-(Cyclobutylmethoxy-methyl)phenyl-, 4-(Cyclopentoxymethyl)phenyl-, 4-(Cyclopentylmethoxy-methyl)phenyl-, 4-(Cyclohexoxymethyl)phenyl-, 4-(Cyclohexylmethoxymethyl)phenyl-, 4-(2-Methoxy-ethyl)phenyl-, 4-(2-Ethoxyethyl)phenyl-, 4-(2-n-Propoxy-ethyl)phenyl-, 4-(2-Isopropoxyethyl)-phenyl-, 4-(2-n-Butoxy-ethyl)phenyl-, 4-(2-(2-Methyl-n-propoxy)ethyl)phenyl-, 4-(2-n-Pentoxyethyl)phenyl-, 4-(2-n-Hexoxy-ethyl)phenyl-, 4-(2-Cyclopropoxy-ethyl)phenyl-, 4-(2-Cyclopropylmethoxy-ethyl)phenyl-, 4-(2-Cyclobutoxyethyl)phenyl-, 4-(2-Cyclobutylmethoxy-ethyl)phenyl-, 4-(2-Cyclopentoxy-ethyl)phenyl-, 4-(2-Cyclopentylmethoxy-ethyl)phenyl-, 4-(2-Cyclohexyloxy-ethyl)phenyl-, 4-(2-Cyclohexylmethoxy-ethyl)phenyl-, 4-(3-Methoxy-propyl)phenyl-, 4-(3-Ethoxy-propyl)phenyl-, 4-(3-n-Propoxypropyl)phenyl-, 4-(3-Isopropoxypropyl)phenyl-, 4-(3-n-Butoxypropyl)phenyl-, 4-(3-(2-Methyl-n-propoxy)propyl)phenyl-, 4-(3-n-Pentoxypropyl)-phenyl-, 4-(3-n-Hexoxypropyl)phenyl-, 4-(3-Cyclopropoxypropyl)phenyl-, 4-(3-Cyclopropylmethoxypropyl)-phenyl-, 4-(3-Cyclobutoxypropyl)phenyl-, 4-(3-Cyclobutylmethoxy-propyl)phenyl-, 4-(3-Cyclopentoxypropyl)-phenyl-, 4-(3-Cyclopentylmethoxy-propyl)phenyl-, 4-(3-Cyclohexoxypropyl)phenyl-, 4-(3-Cyclohexylmethoxy-propyl)phenyl-, 4-((2-Methoxyethoxy)methyl)phenyl-, 4-((2-Ethoxyethoxy)methyl)phenyl-, 4-((2-n-Propoxyethoxy)methyl)phenyl-, 4-((2-Isopropoxyethoxy)methyl)phenyl-, 4-((2-n-Butoxyethoxy)methyl)phenyl-, 4-((2-(2-Methyl-n-propoxy)ethoxy)methyl)phenyl-, 4-((2-n-Pentoxyethoxy)methyl)phenyl-, 4-((2-n-Hexoxyethoxy)-methyl) phenyl-, 4-((2-Cyclopropoxyethoxy)methyl)phenyl-, 4-((2-Cyclopropylmethoxy-ethoxy)methyl)phenyl-, 4-((2-Cyclobutoxyethoxy)methyl)phenyl-, 4-((2-Cyclobutylmethoxy-ethoxy)methyl)phenyl-, 4-((2-Cyclopentoxyethoxy)methyl)phenyl-, 4-((2-Cyclopentylmethoxy-ethoxy)methyl)phenyl-, 4-((2-Cyclohexoxyethoxy)-methyl)phenyl-, 4-((2-Cyclohexylmethoxy-ethoxy)methyl)phenyl-, 4-(2-(2-Methoxyethoxy)ethyl)phenyl-, 4-(2-(2-Ethoxyethoxy)ethyl)phenyl-, 4-(2-(2-n-Propoxyethoxy)ethyl)phenyl-, 4-(2-(2-Isopropoxyethoxy)ethyl)-phenyl-, 4-(2-(2-n-Butoxyethoxy)ethyl)phenyl-, 4-(2-(2-(2-Methyl-n-propoxy)ethoxy)ethyl)phenyl-, 4-(2-(2-n-Pentoxyethoxy)ethyl)phenyl-, 4-(2-(2-n-Hexoxyethoxy)ethyl)phenyl-, 4-(2-(2-Cyclopropoxyethoxy)ethyl)-

phenyl-, 4-(2-(2-Cyclopropylmethoxy-ethoxy)ethyl)phenyl-, 4-(2-(2-Cyclobutoxyethoxy)ethyl)phenyl-, 4-(2-(2-Cyclobutylmethoxy-ethoxy)ethyl)phenyl-, 4-(2-(2-Cyclopentoxyethoxy)ethyl)phenyl-, 4-(2-(2-Cyclopentylmethoxy-ethoxy)ethyl)phenyl-, 4-(2-(2-Cyclohexoxyethoxy)ethyl)phenyl-, 4-(2-(2-Cyclohexylmethoxyethoxy)ethyl)phenyl-, 4-(3-(2-Methoxyethoxy)propyl)phenyl-, 4-(3-(2-Ethoxyethoxy)propyl)phenyl-, 4-(3-(2-n-Propoxyethoxy)propyl)phenyl-, 4-(3-(2-Isopropoxyethoxy)propyl)phenyl-, 4-(3-(2-n-Butoxyethoxy)-propyl)Phenyl-, 4-(3-(2-(2-Methyl-n-propoxy)ethoxy)propyl)phenyl-, 4-(3-(2-n-Pentoxyethoxy)propyl)phenyl-, 4-(3-(2-n-Hexoxyethoxy)propyl)phenyl-, 4-(3-(2-Cyclopropoxyethoxy)propyl)Phenyl-, 4-(3-(2-Cyclopropylmethoxyethoxy)propyl)phenyl-, 4-(3-(2-Cyclobutoxyethoxy)propyl)phenyl-, 4-(3-(2-Cyclobutylmethoxyethoxy)-propyl)phenyl-, 4-(3-(2-Cyclopentoxyethoxy)propyl)phenyl-, 4-(3-(2-Cyclopentylmethoxyethoxy)propyl)-phenyl-, 4-(3-(2-Cyclohexoxyethoxy)propyl)phenyl-, 4-(3-(2-Cyclohexylmethoxyethoxy)propyl)phenyl-, 4-((2-Phenoxyethoxy)methyl)phenyl-, 4-(2-(2-Phenoxyethoxy)ethyl)phenyl-, 4-(3-(2-Phenoxyethoxy)propyl)phenyl-, 2-Chlor-3-methyl-phenyl-, 2-Chlor-4-methyl-phenyl-, 2-Chlor-5-methyl-phenyl-, 2-Chlor-6-methyl-phenyl-, 3-Chlor-2-methyl-phenyl-, 3-Chlor-4-methyl-phenyl-, 3-Chlor-5-methyl-phenyl-, 3-Chlor-6-methyl-phenyl-, 4-Chlor-2-methyl-phenyl-, 4-Chlor-3-methyl-phenyl-, 2-Fluor-3-methyl-phenyl-, 2-Fluor-4-methyl-phenyl-, 2-Fluor-5-methyl-phenyl-, 2-Fluor-6-methyl-phenyl-, 3-Fluor-2-methyl-phenyl-, 3-Fluor-4-methyl-phenyl-, 3-Fluor-5-methyl-phenyl-, 3-Fluor-6-methyl-phenyl-, 4-Fluor-2-methyl-phenyl-, 4-Fluor-3-methyl-phenyl-, 2-Brom-3-methyl-phenyl-, 2-Brom-4-methyl-phenyl-, 2-Brom-5-methyl-phenyl-, 2-Brom-6-methyl-phenyl-, 3-Brom-2-methyl-phenyl-, 3-Brom-4-methyl-phenyl-, 3-Brom-5-methyl-phenyl-, 3-Brom-6-methyl-phenyl-, 4-Brom-2-methyl-phenyl- oder 4-Brom-3-methyl-phenylgruppe,

für A die der Ethylen-, n-Propylen-, n-Butylen-, $\alpha$-Methyl-ethylen-, $\alpha$-Methyl-n-propylen-, $\alpha$-Methyl-n-butylen-, $\alpha,\alpha$-Dimethyl-ethylen-, $\alpha,\alpha$-Dimethyl-n-propylen-, $\alpha,\alpha$-Dimethyl-n-butylen-, $\alpha,\alpha$-Diethyl-n-ethylen-, $\alpha,\alpha$-Diethyl-n-propylen-, $\alpha,\alpha$-Diethyl-n-butylen-, $\alpha$-Methyl-$\alpha$-ethyl-ethylen-, $\alpha$-Methyl-$\alpha$-ethyl-n-propylen-, $\alpha$-Methyl-$\alpha$-ethyl-n-butylen-, $\alpha$-Methyl-$\alpha$-n-propyl-ethylen-, $\alpha$-Methyl-$\alpha$-n-propyl-n-propylen-oder $\alpha$-Ethyl-$\alpha$-n-propyl-n-propylengruppe und

für $R_2$ die der 3-(3(2H)-Pyridazinon-6-yl)phenoxy-, 4-(3(2H)-Pyridazinon-6-yl)phenoxy-, 3-(4,5-Dihydro-3(2H)-pyridazinon-6-yl)phenoxy-, 3-(4,5-Dihydro-5-methyl-3(2H)-pyridazinon-6-yl)phenoxy-, 3-(4,5-Dihydro-5-ethyl-3(2H)-pyridazinon-6-yl)phenoxy-, 3-(4,5-Dihydro-5-isopropyl-3(2H)-pyridazinon-6-yl)phenoxy-, 4-(4,5-Dihydro-3(2H)-pyridazinon-6-yl)phenoxy-, 4-(4,5-Dihydro-5-methyl-3(2H)-pyridazinon-6-yl)phenoxy-, 4-(4,5-Dihydro-5-ethyl-3(2H)-pyridazinon-6-yl)phenoxy-, 4-(4,5-Dihydro-5-isopropyl-3(2H)-pyridazinon-6-yl)phenoxy-, 2-Methyl-3-(3(2H)-pyridazinon-6-yl)phenoxy-, 2-Methyl-4-(3(2H)-pyridazinon-6-yl)phenoxy-, 2-Methyl-3-(4,5-dihydro-3(2H)-pyridazinon-6-yl)phenoxy-, 2-Methyl-3-(4,5-dihydro-5-methyl-3(2H)-pyridazinon-6-yl)phenoxy-, 2-Methyl-3-(4,5-dihydro-5-ethyl-3(2H)-pyridazinon-6-yl)phenoxy-, 2-Methyl-3-(4,5-dihydro-5-isopropyl-3(2H)-pyridazinon-6-yl)phenoxy-, 2-Methyl-4-(4,5-dihydro-3(2H)-pyridazinon-6-yl)phenoxy-, 2-Methyl-4-(4,5-dihydro-5-methyl-3(2H)-pyridazinon-6-yl)phenoxy-, 2-Methyl-4-(4,5-dihydro-5-ethyl-3(2H)-pyridazinon-6- yl)-phenoxy-, 2-Methyl-4-(4,5-dihydro-5-isopropyl-3(2H)-pyridazinon-6-yl)phenoxy-, 2-Chlor-4-(3(2H)-pyridazinon-6-yl)phenoxy-, 2-Chlor-4-(4,5-dihydro-3(2H)-pyridazinon-6-yl)phenoxy-, 2-Chlor-4-(4,5-dihydro-5-methyl-3(2H)-pyridazinon-6-yl)phenoxy-, 2-Chlor-4-(4,5-dihydro-5-ethyl-3(2H)-pyridazinon-6-yl)phenoxy-, 2-Chlor-4-(4,5-dihydro-5-isopropyl-3(2H)-pyridazinon-6-yl)phenoxy-, 5-(3(2H)-Pyridazinon-6-yl)benzoxazol-(2)-imino-, 5-(4,5-Dihydro-3(2H)-pyridazinon-6-yl)benzoxazol-(2)-imino-, 5-(4,5-Dihydro-5-methyl-3(2H)-pyridazinon-6-yl)benzoxazol-(2)-imino-, 5-(4,5-Dihydro-5-ethyl-3(2H)-pyridazinon-6-yl)benzoxazol-(2)-imino-, 5-(4,5-Dihydro-5-isopropyl-3(2H)-pyridazinon-6-yl)benzoxazol-(2)-imino-, 5-(3(2H)-Pyridazinon-6-yl)-benzimidazol-1-yl-, 5-(4,5-Dihydro-3(2H)-pyridazinon-6-yl)benzimidazol-1-yl-, 5-(4,5-Dihydro-5-methyl-3(2H)-pyridazinon-6-yl)benzimidazol-1-yl-, 5-(4,5-Dihydro-5-ethyl-3(2H)-pyridazinon-6-yl)benzimidazol-1-yl-, 5-(4,5-Dihydro-5-isopropyl-3(2H)-pyridazinon-6-yl)- benzimidazol-1-yl-, 5-(3(2H)-Pyridazinon-6-yl)benzimidazol2-yl-, 5-(4,5-Dihydro-3(2H)-pyridazinon-6-yl)benzimidazol2-yl-, 5-(4,5-Dihydro-5-methyl-3(2H)-pyridazinon-6-yl)-benzimidazol-2-yl-, 5-(4,5-Dihydro-5-ethyl-3(2H)-pyridazinon-6-yl)benzimidazol-2-yl-, 5-(4,5-Dihydro-5-isopropyl-3(2H)-pyridazinon-6-yl)benzimidazol-2-yl-, 5-(3(2H)Pyridazinon-6-yl)-2-methyl-benzimidazol-1-yl-, 5-(4,5-Dihydro-3(2H)-pyridazinon-6-yl)-2-methyl-benzimidazol-1-yl-, 5-(4,5-Dihydro-5-methyl-3(2H)-pyridazinon-6-yl)-2-methylbenzimidazol-1-yl-, 5-(4,5-Dihydro-5-ethyl-3(2H)-pyridazinon-6-yl)-2-methyl-benzimidazol-1-yl-, 5-(4,5-Dihydro-5-isopropyl-3(2H)-pyridazinon-6-yl)-2-methyl-benzimidazol-1-yl-, 5-(4,5-Dihydro-3(2H)-pyridazinon-6-yl)-2-hydroxy-benzimidazol-1-yl-, 5-(4,5-Dihydro-5-methyl-3(2H)-pyridazinon-6-yl)-2-hydroxy-benzimidazol-1-yl-, 5-(4,5-Dihydro-5-ethyl-3(2H)-pyridazinon-6-yl)-2-hydroxy-benzimidazol-1-yl-, 5-(4,5-Dihydro-5-isopropyl-3(2H)-pyridazinon-6-yl)-2-hydroxy-benzimidazol-1-yl-, 5-(3(2H)-Pyridazinon-6-yl)benztriazol-1-yl-, 5-(4,5-Dihydro-3(2H)-pyridazinon-6-yl)benztriazol-1-yl-, 5-(4,5-Dihydro-5-methyl-3(2H)-pyridazinon-6-yl)benztriazol- 1-yl-, 5-(4,5-Dihydro-5-ethyl-3(2H)-pyridazinon-6-yl)benztriazol-1-yl-, 5-(4,5-Dihydro-5-isopropyl-3(2H)-pyridazinon-6-yl)benztriazol-1-yl-, 4-(3(2H)-Pyridazinon-6-yl)phenylsulfonyl-, 4-(4,5-Dihydro-3(2H)-pyridazinon-6-yl)Phenylsulfonyl-, 4-(4,5-Dihydro-5-methyl-3(2H)-pyridazinon-6-yl)-phenylsulfonyl-, 4-(4,5-Dihydro-5-ethyl-3(2H)-Pyridazinon-6-yl)phenylsulfonyl-, 4-(4,5-Dihydro-5-isopropyl-3-

(2H)-pyridazinon-6-yl)phenylsulfonyl-, 4-(3(2H)-Pyridazinon-6-yl)phenyl-, 4-(4,5-Dihydro-3(2H)-pyridazinon-6-yl)phenyl-, 4-(4,5-Dihydro-5-methyl-3(2H)-pyridazinon-6-yl)phenyl-, 4-(4,5-Dihydro-5-ethyl-3(2H)-pyridazinon-6-yl)phenyl-, 4-(4,5-Dihydro-5-isopropyl-3(2H)-Pyridazinon-6-yl)phenyl-, 4-(3(2H)-Pyridazinon-6-yl)-phenylmethylcarbonylamino-, 4-(4,5-Dihydro-3(2H)-pyridazinon-6-yl)Phenylmethylcarbonylamino-, 4-(4,5-Dihydro-5-methyl-3(2H)-pyridazinon-6-yl)phenylmethylcarbonylamino-, 4-(4,5-Dihydro-5-ethyl-3(2H)-pyridazinon-6-yl)phenylmethylcarbonylamino- oder 4-(4,5-Dihydro-5-isopropyl-3(2H)-pyridazinon-6-yl)-phenylmethylcarbonylaminogruppe in Betracht.

Bevorzugte Verbindungen der obigen Formel sind diejenigen, in denen

$R_1$ eine Naphthyl-, 3,4-Dihydro-carbostyril-(5)-, Allylphenyl-, Allyloxyphenyl-, Cyanophenyl-, Aminocarbonylmethylphenyl- oder Chlor-methylphenylgruppe, eine Alkylsulfonyloxyphenylgruppe mit 1 bis 4 Kohlenstoffatomen im Alkylteil oder eine in 4-Stellung durch einen 2-Alkoxyethyl-, 2-Cycloalkylmethoxyethyl-, 2-Alkoxyethoxymethyl-, 2-Cycloalkoxyethoxymethyl-, 2-Cycloalkylmethoxyethoxymethyl- oder 2-Phenoxyethoxymethylrest substituierte Phenylgruppe, in welcher der Alkoxyteil jeweils 1 bis 4 Kohlenstoffatome und der Cycloalkyl-oder Cycloalkoxyteil jeweils 3 oder 4 Kohlenstoffatome enthalten kann,

A eine gegebenenfalls in α-Stellung zu dem benachbarten Stickstoffatom durch eine Methylgruppe mono- oder disubstituierte n-Alkylengruppe mit 2 bis 4 Kohlenstoffatomen und

$R_2$ eine Gruppe der Formel

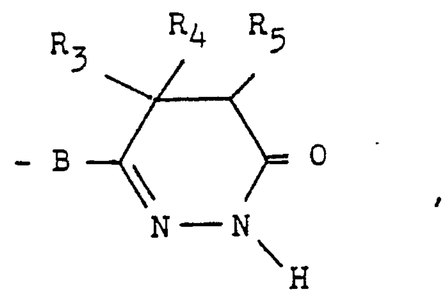

in welcher

$R_3$ ein Wasserstoffatom oder eine Methylgruppe,

$R_4$ und $R_5$ jeweils ein Wasserstoffatom oder zusammen eine weitere Kohlenstoff-Kohlenstoffbindung und

B eine Gruppe der Formeln

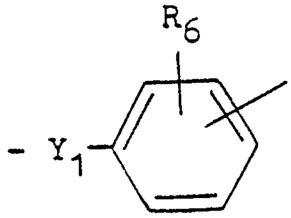

,

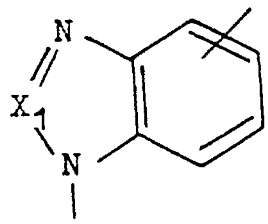

oder

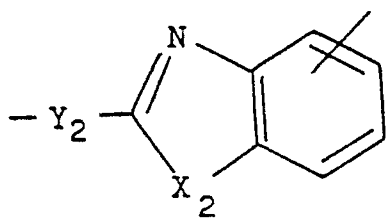

darstellen, in welchen
$R_6$ ein Wasserstoff-, Fluor-, Chlor- oder Bromatom oder eine Methylgruppe,
$Y_1$ eine Bindung, ein Sauerstoffatom oder eine -NH-CO-$CH_2$-Gruppe, wobei die -$CH_2$-Gruppe mit dem Phenylkern verbunden ist,
$X_1$ ein Stickstoffatom, eine gegebenenfalls durch eine Hydroxy- oder Methylgruppe substituierte Methingruppe,
$X_2$ ein Sauerstoffatom oder eine Iminogruppe und
$Y_2$ eine Bindung oder eine Iminogruppe bedeuten, deren Tautomere, deren Enantiomere und deren Säureadditionssalze.

Besonders bevorzugte Verbindungen der obigen Formel I sind jedoch diejenigen, in denen
$R_1$ eine Naphthyl-, 3,4-Dihydro-carbostyril-(5)-, Allylphenyl-, Allyloxyphenyl- oder Chlor-methylphenylgruppe oder eine in 4-Stellung durch einen 2-Alkoxyethyl-, 2-Cycloalkylmethoxyethyl-, 2-Alkoxyethoxymethyl-, 2-Cycloalkoxyethoxymethyl-, 2-Cycloalkylmethoxyethoxymethyl- oder 2-Phenoxyethoxymethylrest substituierte Phenylgruppe, in welcher der Alkoxyteil jeweils 1 bis 3 Kohlenstoffatome und der Cycloalkyl- oder Cycloalkoxyteil jeweils 3 oder 4 Kohlenstoffatome enthalten kann,
A eine gegebenenfalls in α-Stellung zu dem benachbarten Stickstoffatom durch Methylgruppen disubstituierte n-Alkylengruppe mit 2 bis 4 Kohlenstoffatomen und
$R_2$ eine Gruppe der Formel

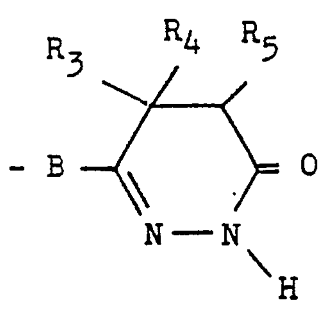

,

in welcher
$R_3$ ein Wasserstoffatom oder eine Methylgruppe,
$R_4$ und $R_5$ jeweils ein Wasserstoffatom oder zusammen eine weitere Kohlenstoff-Kohlenstoffbindung und
B eine Gruppe der Formeln

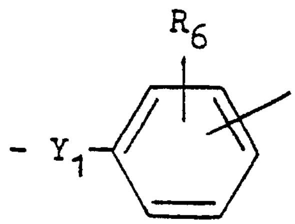 ,

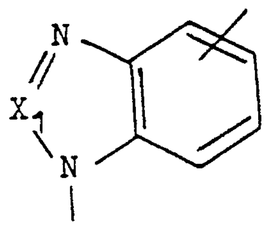 oder

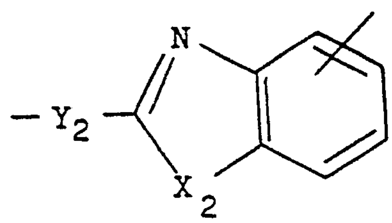

darstellen, in welchen
$R_6$ eine Methylgruppe, ein Wasserstoff- oder Chloratom,
$Y_1$ ein Sauerstoffatom,
$X_1$ eine Methingruppe,
$X_2$ ein Sauerstoffatom oder eine Iminogruppe und
$Y_2$ eine Bindung oder eine Iminogruppe bedeuten, deren Tautomere, deren Enantiomere und deren Säureadditionssalze.

Erfindungsgemäß erhält man die Verbindungen der Formel I durch Umsetzung einer Verbindung der Formel
$R_1$-O-$CH_2$-CHOH-$CH_2$-Z ,(II)
in der
$R_1$ wie eingangs definiert ist und
Z eine nukleophile Austrittsgruppe wie ein Halogenatom oder einen Sulfonsäureesterrest, z.B. ein Chlor-, Brom- oder Jodatom, eine Methylsulfonyloxy- oder p-Toluolsulfonyloxygruppe, oder
Z zusammen mit dem Wasserstoffatom der benachbarten Hydroxygruppe eine Sauerstoff-Kohlenstoff-Bindung bedeutet, mit einem Amin der Formel
$NH_2$-A-$R_2$ ,(III)
in der
A und $R_2$ wie eingangs definiert sind.

Die Umsetzung wird vorzugsweise in einem Lösungsmittel wie Ethanol, Isopropanol, Tetrahydrofuran, Dioxan, Benzol, Toluol, Dimethylformamid oder Dimethylsulfoxid gegebenenfalls in Gegenwart eines säurebindenden Mittels wie Triethylamin oder Pyridin, welche gleichzeitig als Lösungsmittel verwendet werden können, bei Temperaturen zwischen 0 und 100°C, vorzugsweise jedoch bei Temperaturen zwischen 25 und 80°C, durchgeführt.

Die erhaltenen Verbindungen der Formel I lassen sich, da diese mindestens ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure oder D- oder L-Camphersäure.

Außerdem können die erhaltenen Verbindungen der Formel I gewünschtenfalls in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der Formel II und III erhält man nach literaturbekannten Verfahren.

So erhält man beispielsweise eine Verbindung der Formel II durch Umsetzung eines entsprechenden Phenols mit einem entsprechenden Epihalogenhydrin oder mit einem entsprechend in 1- und 3-Stellung substituierten 2-Hydroxypropan.

Eine Verbindung der Formel III erhält man durch Abspaltung von einem durch einen entsprechenden Schutzrest geschützten Amin der Formel III, wobei man die hierfür erforderliche Ausgangsverbindung entweder durch Umsetzung einer entsprechenden 4-Oxobuttersäure mit Hydrazin, durch Umsetzung eines entsprechenden 2-Methylthio-benzoxazols (siehe EP-A-0.034.743) mit einem entsprechenden Amin oder durch Alkylierung eines entsprechenden Phenols, Thiophenols, Benzimidazols oder Benztriazols erhält, wobei die so erhaltene Thioverbindung anschließend mittels Oxidation in das entsprechende Sulfon übergeführt wird.

Wie bereits eingangs erwähnt, weisen die neuen 2-Hydroxy-n-propylamine der Formel I, deren Tautomere, deren Enantiomeren und deren physiologisch verträglichen Säureadditionssalze wertvolle pharmakologische Eigenschaften auf, insbesondere eine gleichzeitige cardiotone und ß-blockierende Wirkung.

Beispielsweise wurden die Verbindungen

A = 6-[1-[2-[3-(4-(2-Cyclobutylmethoxy-ethyl)phenoxy)-2-hydroxy-propylamino]ethyl]benzimidazol-5-yl]-4,5-dihydro-3(2H)-pyridazinon,

B = 6-[1-[2-[3-(4-(2-Cyclopropylmethoxy-ethyl)phenoxy)-2-hydroxy-propylamino]ethyl]benzimidazol-5-yl]-4,5-dihydro-3(2H)-pyridazinon,

C = 6-[4-[2-[3-(4-(2-Cyclopropylmethoxy-ethyl)phenoxy)-2-hydroxy-propylamino]ethoxy]phenyl]-4,5-dihydro-3(2H)-pyridazinon,

D = 6-[4-[2-[3-(4-(2-Cyclopropylmethoxyethyl)phenoxy)-2-hydroxy-propylamino]ethoxy]phenyl]-3(2H)-pyridazinon,

E = 6-[4-[2-[3-(4-(2-Cyclobutylmethoxy-ethyl)phenoxy)-2-hydroxy-propylamino]ethoxy]phenyl]-4,5-dihydro-3(2H)-pyridazinon,

F = 6-[4-[2-[3-(4-((2-Isopropoxyethoxy)methyl)phenoxy)-2-hydroxy-propylamino]ethoxy]phenyl]-4,5-dihydro-3(2H)-pyridazinon,

G = 6-[4-[2-[3-(4-(2-Isopropoxyethoxymethyl)phenoxy)-2-hydroxypropylamino]ethoxy]phenyl]-3(2H)-pyridazinon,

H = 6-[1-[3-[3-(4-(2-Isobutoxyethyl)phenoxy)-2-hydroxypropylamino]propyl]benzimidazol-5-yl]-4,5-dihydro-3(2H)-pyridazinon,

I = 6-[1-[2-[3-(4-(2-Isobutoxyethyl)phenoxy)-2-hydroxypropylamino]-2,2-dimethyl-ethyl]benzimidazol-5-yl]-4,5-dihydro-3(2H)-pyridazinon,

K .= 6-[4-[3-[3-(4-(2-Isopropoxyethoxy-methyl)phenoxy)-2-hydroxy-propylamino]propoxy]-3-chlor-phenyl]-4,5-dihydro-3(2H)-pyridazinon,

L = 6-[4-[2-[3-(4-(2-Isopropoxyethoxy-methyl)phenoxy)-2-hydroxy-propylamino]ethoxy]-3-chlor-phenyl]-4,5-dihydro-3(2H)-pyridazinon,

M = 6-[4-[2-[3-(4-(2-Cyclopropylmethoxy-ethyl)phenoxy)-2-hydroxy-propylamino]ethoxy]-3-methyl-phenyl]-4,5-dihydro-3(2H)-pyridazinon,

N = 6-[4-[2-[3-(4-(2-Isopropoxyethoxy-methyl)phenoxy)-2-hydroxy-propylamino]ethoxy]-3-methyl-phenyl]-4,5-dihydro-3(2H)-pyridazinon,

0 = 6-[4-[3-[3-(4-(2-Cyclopropylmethoxy-ethyl)phenoxy)-2-hydroxy-propylamino]propoxy]-3-methyl-phenyl]-4,5-dihydro-3(2H)-pyridazinon,

P = 6-[4-[3-[3-(4-(2-Cyclobutylmethoxy-ethyl)Phenoxy)-2-hydroxy-propylamino]propoxy]-3-methyl-phenyl]-4,5-dihydro-3(2H)-pyridazinon und

Q = 6-[4-[2-[3-(4-(2-Isopropoxyethoxy-methyl)phenoxy)-2-hydroxy-propylamino]ethoxy]-3-methyl-phenyl]-3(2H)-pyridazinon auf ihre biologischen Eigenschaften wie folgt untersucht:

1. Affininät zu ß-Adrenorezeptoren:

Die Hemmwirkung auf die Bindung von $^3$H-CGP 12177 wurde an Rezeptorpräparationen aus Ratten-Herzen ($\beta_1$) und Ratten-Lunge ($\beta_2$) geprüft. Hierbei wurde der Bindungsassay mit einigen Modifikationen nach der Methode von Dämmgen et al. (Drug. Res. 35, 383-390 (1985)) durchgeführt. Der Inkubationsansatz enthielt Aliquote der Membranpräparation in Tris-Puffer, steigende Konzentrationen des potentiellen Antagonisten sowie 200 pM $^3$H-CGP 12177. Die Trennung von gebundenen und freien Radioliganden erfolgte nach Ablauf der Inkubationszeit durch rasche Filtration durch Glasfiber-Filter. Die Filter-gebundene Radioaktivität wurde in einen ß-Counter bei einer Zählausbeute von etwa 52 % ermittelt. Die unspezifische Bindung wurde in Gegenwart von $10^{-5}$ M Propranolol ermittelt.

| Substanz | $IC_{50}$ (nM) | |
|---|---|---|
| | $\beta_1$ | $\beta_2$ |
| A | 26 | 940 |
| B | 34 | 2300 |
| C | 29 | 1200 |
| D | 22 | 1000 |
| E | 15 | 1100 |
| F | 24 | 7200 |
| G | 53 | 4400 |
| H | 10 | 19 |
| I | 12 | 65 |
| K | 10 | 350 |
| L | 7.6 | 600 |
| M | 0.5 | 100 |
| N | 2.6 | 520 |
| O | 59 | 66 |
| P | 6.0 | 220 |
| Q | 2.0 | 210 |

2. Kardiotonische Wirkung mit antiadrenergen Aktivität:

Meerschweinchen (Dunkin-Hartley-Pirbright, männlich, 450-600 g) werden mit Hexobarbital-Natrium (150 mg/kg i.p.) narkotisiert. Nach Eintreten der Narkose wird eine Trachealkanüle gelegt und die Tiere despinalisiert (van Meel, J. Pharmacol. Methods 31 (1), 1-11 (1985)). Die Tiere werden dann sofort künstlich mit einer Atempumpe beatmet. Linker ventrikulärer Druck (LVP) wird mit einem Millar PR-249 Kathetertipmanometer, das über die linke Arteria Carotis in den linken Ventrikel geschoben wurde, gemessen. Dieses Drucksignal wird elektronisch differenziert (LV-dP/dtmax) und als Parameter für die Inotropie benutzt. Arterieller Blutdruck wird durch eine Kanüle in der rechten Arteria Carotis mittels eines Bell & Howell Druckaufnehmer registriert. Zusätzlich wird die Herzfrequenz gemessen. Substanzen werden über eine Kanüle in der Vena jugularis appliziert.

Test-Substanzen werden kumulativ in vier Dosierungen (0,1 bis 3 mg/kg) drei Tieren appliziert und die Effekte auf Blutdruck, Herzfrequenz und LV-dP/dtmax gemessen. Drei Minuten nach der letzten Dosis der Test-Substanz wird dann Isoprenalin in steigenden Dosen intravenös appliziert. So wird eine kumulative Dosis-Wirkungsbeziehung für Isoprenalin in Gegenwart der Test-Substanz erhalten. Messparameter sind Steigerung der Herzfrequenz und LV-dP/dtmax. Diese Dosis-Wirkungskurven werden mit Standardkurven für Isoprenalin ohne Test-Substanzen verglichen. Grafisch werden die Rechtsverschiebungen der Dosis-Wirkungskurven von Isoprenalin durch Test-Substanzen ermittelt und entsprechende pA2-Werte für die Test-Substanzen berechnet (siehe Tenakin in "Pharmakologic Analysis of Drug Receptor Interaction", Rawen Press, 211-212 (1987)).

Die Tabelle zeigt die Effekte der Test-Substanzen nach der Dosis von 3 mg/kg i.v. auf LV-dP/dtmax und Herzfrequenz und die berechneten pA2-Werte der Test-Substanzen gegenüber der inotropen (pA2 Inotropie) und herzfrequenzsteigernden (pA2 Herzfrequenz) Wirkung von Isoprenalin.

Die nachfolgende Tabelle enthält die Mittelwerte aus 2 bis 3 Versuchen:

| Substanz | Effekte auf LV-dP/dtmax % Zunahme | Effekt auf Herzfrequenz in Schläge/Min | pA2 Inotropie | pA2 Herzfrequenz |
|---|---|---|---|---|
| A | + 50 | - 23 | 6.30 | 8.20 |
| B | + 51 | - 47 | 6.83 | 6.95 |
| C | + 25 | - 51 | 6.76 | 7.22 |
| D | + 51 | - 49 | 6.67 | 7.24 |
| E | + 49 | - 71 | 6.19 | 7.23 |
| F | + 37 | - 94 | 7.14 | 6.96 |
| G | + 38 | - 36 | 6.32 | 6.38 |
| H | + 10 | - 90 | 8.28 | > 9.0 |
| I | + 58 | - 59 | 7.17 | 7.89 |
| K | + 11 | -121 | 6.56 | 7.52 |
| L | + 66 | - 51 | 6.23 | 6.77 |
| M | + 51 | - 64 | 7.05 | 8.20 |
| N | + 34 | - 94 | 7.34 | 8.34 |
| O | + 38 | -116 | 6.44 | 7.70 |
| P | + 69 | - 61 | 5.31 | 6.45 |

Die vorstehend erwähnten Verbindungen weisen bis zu einer Dosis von 4,4 mg/kg i.v. keine negativ inotropen Wirkungen auf, außerdem konnten keine Herzrhythmusstörungen beobachtet werden. Die neuen Verbindungen sind daher in therapeutischen Dosen gut verträglich.

Aufgrund ihrer pharmakologischen Eigenschaften eignen sich die neuen Verbindungen und deren physiologisch verträgliche Additionssalze zur Behandlung der myocardialen Ischämie (Angina), zur Behandlung der Herzinsuffizienz, zur Prävention der Herzinsuffizienzprogression nach Myocard-Infarkt und zur Behandlung der Hypertonie.

Die zur Erzielung einer entsprechenden Wirkung erforderliche Dosierung beträgt zweckmäßigerweise bei intravenöser Gabe 20 bis 100 mg, vorzugsweise 30 bis 75 mg, und bei oraler Gabe 50 bis 200 mg, vorzugsweise 75 bis 150 mg, jeweils 1 bis 2 x täglich. Hierzu lassen sich die erfindungsgemäß hergestellten Verbindungen der Formel, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zübereitungen wie Tabletten, Dragees, Kapseln, Pulver, Suspensionen oder Zäpfchen einarbeiten.

Die nachfolgenden Beispiele sollen die Erfindung näher erläutern:

## Beispiel I

### 1-(1-Naphthyloxy)-2,3-epoxypropan

14,1 g (100 mMol) 1-Naphthol und 11,2 g (100 mMol) Kaliumtert.butylat werden bei 50° C in 50 ml Dimethylsulfoxid gelöst. Unter Rühren werden danach bei Raumtempertur 15,0 g (110 mMol) Epibromhydrin zugetropft, wobei sich das Gemisch allmählich auf ca. 50° C erwärmt. Nach zwei Stunden werden 200 ml Wasser hinzugegeben und dreimal mit je 50 ml Essigsäureethylester extrahiert. Die vereinigten organischen Phasen werden mit 100 ml Wasser gewaschen, über Natriumsulfat getrocknet und eingeengt. Das so erhaltene Rohprodukt wird durch Vakuumdestillation gereinigt ($Kp_{0,1mm}$: 108-110° C).

Ausbeute: 16,1 g (80 % der Theorie)

Analog werden folgende Epoxide erhalten:

1-(4-Aminocarbonylmethyl-phenoxy)-2,3-epoxypropan

$R_F$-Wert: 0,68 (Kieselgel, Dichlormethan/Methanol = 9:1)

Schmelzpunkt: 172-173 °C
(3,4-Dihydrochinolin-2-on-5-yl)-2,3-epoxy-1-propyläther
$R_F$-Wert: 0,66 (Kieselgel, Dichlormethan/Methanol = 9:1)
1-(2-Allyloxy-phenoxy)-2,3-epoxypropan
$R_F$-Wert: 0,88 (Kieselgel, Dichlormethan)
1-(2-Cyano-phenoxy)-2,3-epoxypropan
$R_F$-Wert: 0,86 (Kieselgel, Dichlormethan/Methanol = 9:1)
1-(2-Chlor-5-methyl-phenoxy)-2,3-epoxypropan
$R_F$-Wert: 0,85 (Kieselgel, Dichlormethan)
1-(2-Allyl-phenoxy)-2,3-epoxypropan
$R_F$-Wert: 0,92 (Kieselgel, Dichlormethan)
1-[4-(2-Cyclobutylmethoxy-ethyl)phenoxy]-2,3-epoxypropan
$R_F$-Wert: 0,43 (Kieselgel, Dichlormethan/Methanol = 50:1)
1-[4-(2-Isobutoxy-ethyl)phenoxy]-2,3-epoxypropan
$R_F$-Wert: 0,62 (Kieselgel, Dichlormethan/Methanol = 19:1)
1-[4-(2-Cypropylmethoxy-ethyl)phenoxy]-2,3-epoxypropan
$R_F$-Wert: 0,31 (Kieselgel, Dichlormethan)
1-(4-Methansulfonyloxy-phenoxy)-2,3-epoxypropan
$R_F$-Wert: 0,66 (Kieselgel, Dichlormethan/Ethanol = 50:1)
1-(4-Butansulfonyloxy-phenoxy)-2,3-epoxypropan
$R_F$-Wert: 0,42 (Kieselgel, Dichlormethan/Ethanol = 50:1)
1-(4-Isopropansulfonyloxy-phenoxy)-2,3-epoxypropan
$R_F$-Wert: 0,38 (Kieselgel, Dichlormethan/Ethanol = 50:1)
1-(4-(2-Isopropoxyethoxy)methyl-phenoxy)-2,3-epoxypropan
$R_F$-Wert: 0,68 (Kieselgel, Dichlormethan/Ethanol = 19:1)
1-[4-(2-Phenoxyethoxymethyl)phenoxy]-2,3-epoxypropan
$R_f$-Wert: 0,70 (Kieselgel, Dichlormethan/Ethanol = 19:1)
1-[4-(2-n-Butyloxyethoxymethyl)phenoxy]-2,3-epoxypropan
$R_f$-Wert: 0,69 (Kieselgel, Dichlormethan/Ethanol = 19:1)
1-[4-(2-Methoxyethoxy-methyl)phenoxy]-2,3-epoxypropan
$R_f$-Wert: 0,78 (Kieselgel, Dichlormethan/Methanol = 30:1)

## Beispiel II

### 6-[4-(3-Phthalimidopropylsulfonyl)phenyl]-4,5-dihydro-3(2H)-pyridazinon

34 g (78,2 mMol) 4-Oxo-4-[4-(3-phthalimidopropylsulfonyl)phenyl]buttersäuremethylester werden in einer Lösung von 6 ml (120 mMol) Hydrazinhydrat (99 %) in 350 ml Eisessig zwei Stunden lang zum Rückfluß erhitzt. Danach wird das Gemisch auf 2,5 1 Eiswasser gegossen, das ausgefallene Produkt abgesaugt und im Umlufttrockenschrank bei 80 °C getrocknet.
Ausbeute: 96,5 % der Theorie,
Schmelzpunkt: 205-207 °C

## Beispiel III

### 6-[1-(2-Acetylaminoethyl)benzimidazol-5-yl]-3(2H)-pyridazinon

14,2 g (49,4 mMol) 6-(3-Amino-4-acetylaminoethylaminophenyl)-3(2H)-pyridazinon werden in 60 ml Ameisensäure eine Stunde lang zum Rückfluß erhitzt. Danach wird im Vakuum die überschüssige Ameisensäure abdestilliert, der Rückstand mit 50 ml Eiswasser versetzt, dann mit konzentrierter Ammoniaklösung auf pH 8-9 eingestellt. Das ausgefallene Produkt wird abgesaugt, mit ca. 50 ml kaltem Wasser, dann mit 50 ml Äther nachgewaschen und getrocknet.
Ausbeute: 54 % der Theorie,
Schmelzpunkt: > 250 °C

Beispiel IV

6-[1-(2-Acetaminoethyl)-2-hydroxy-benzimidazol-5-yl]-4,5-dihydro-3(2H)-pyridazinon

Zu einer Lösung von 580 mg (2,0 mMol) 6-(3-Amino-4-acetylaminoethylamino-phenyl)-4,5-dihydro-3-(2H)-pyridazinon in 50 ml Dimethylformamid werden bei 50 °C 325 mg (2,0 mMol) Carbonyldiimidazol hinzugefügt. Nach 45 Minuten bei 50 °C wird zur Trockne eingedampft und der Rückstand mit 10 ml Wasser versetzt. Das ausgefallene Produkt wird abgesaugt, mit 5 ml Wasser gewaschen und getrocknet.
Ausbeute: 79 % der Theorie,
Schmelzpunkt: > 250 °C

Beispiel V

6-[1-(2-Acetaminoethyl)benztriazol-5-yl]-4,5-dihydro-3(2H)-pyridazinon

15,98 g (55,3 mMol) 6-(3-Amino-4-acetaminoethylamino-phenyl)-4,5-dihydro-3(2H)-pyridazinon werden in 300 ml 2n Schwefelsäure gelöst, dann auf 2-5 °C gekühlt und eine Lösung von 4,2 g (61 mMol) Natriumnitrit in 70 ml Wasser unter Rühren langsam zugetropft. Nach ca. einer Stunde wird das Kühlbad entfernt und das Gemisch bei Raumtemperatur eine weitere Stunde gerührt. Der ausgefallene Feststoff wird abgesaugt, in 100 ml einer 5%igen Natriumbicarbonatlösung eingerührt und nach 10 Minuten erneut abgesaugt.
Ausbeute: 49 % der Theorie,
Schmelzpunkt: 255-257 °C

Beispiel VI

5-Methyl-6-[2-(2-phthalimidoethyl)benzimidazol-5-yl]-4,5-dihydro-3(2H)-pyridazinon

120 g (286 mMol) 5-Methyl-6-[3-amino-4-(2-phthalimidopropionylamido)phenyl]-4,5-dihydro-3(2H)-pyridazinon werden eine Stunde lang in 1,2 1 Eisessig zum Rückfluß erhitzt. Das Gemisch wird dann in 1 1 Wasser eingerührt und die dabei ausgefallenen Verunreinigungen abfiltriert. Das Filtrat wird anschließend am Rotationsverdampfer bis zur Trockne eingedampft, der feste Rückstand mit 200 ml 2n Ammoniaklösung verrieben. Dann wird der Feststoff abgesaugt und aus Methanol umkristallisiert.
Ausbeute: 71 % der Theorie,
Schmelzpunkt: 148-150 °C

Beispiel VII

5-Methyl-6-[2-(4-Aminobutylamino)benzoxazol-5-yl]-4,5-dihydro-3(2H)-pyridazinon

4,1 g (15,0 mMol) 5-Methyl-6-(2-methylthio-benzoxazol-5-yl)-4,5-dihydro-3(2H)-pyridazinon und 15 ml 1,4-Diaminobutan werden 45 Minuten lang auf 60 °C erhitzt, anschließend in 200 ml Wasser eingerührt und zur Kristallisation bei Raumtemperatur stehen gelassen. Nach ca. 30 Minuten wird das Produkt abgesaugt, mit ca. 50 ml Wasser gewaschen und bei 60 °C im Umlufttrockenschrank getrocknet.
Ausbeute: 89 % der Theorie,
Schmelzpunkt: 184-187 °C

Beispiel VIII

6-[4-((2-tert.Butoxycarbonylamino)ethylaminocarbonylmethyl)phenyl]-4,5-dihydro-3(2H)-pyridazinon

2,3 g (10,0 mMol) 6-(4-Carboxymethyl-phenyl)-4,5-dihydro-3(2H)-pyridazinon werden in 30 ml Dimethylformamid gelöst, 1,8 g (11,0 mMol) Carbonyldiimidazol hinzugegeben und 30 Minuten lang bei Raumtempertur gerührt. Dann werden 1,2 g (7,3 mMol) 2-tert.Butoxycarbonylaminoethylamin hinzugefügt und eine weitere Stunde gerührt. Das Reaktionsgemisch wird dann in ca. 1 1 Wasser eingerührt und zur Kristallisation über Nacht stehengelassen. Das ausgefallene Produkt wird abgesaugt, mit ca. 30 ml Wasser nachgewaschen und im Umlufttrockenschrank getrocknet.
Ausbeute: 52 % der Theorie,
Schmelzpunkt: 226-228° C

Beispiel IX

6-[4-((2-Aminoethyl)aminocarbonylmethyl)phenyl]-4,5-dihydro-3(2H)-pyridazinon

Eine Mischung von 6,8 g (18 mMol) 6-[4-((2-tert.Butoxycarbonylaminoethyl)aminocarbonylmethyl)-phenyl]-4,5-dihydro-3(2H)-pyridazinon, 25 ml Ethanol und 50 ml 2n Salzsäure wird ca. zwei Minuten lang unter Rückfluß erhitzt, danach das Ethanol abdestilliert, die wäßrige Lösung dann mit konzentrierter Ammoniaklösung alkalisch gestellt und schließlich das ausgefallene Produkt abgesaugt. Die Reinigung erfolgt durch Chromatographie über 200 g Kieselgel (Elutionsmittel: Dichlormethan/Methanol/Ammoniak = 70:30:1).
Ausbeute: 69 % der Theorie,
Schmelzpunkt: 193-196° C

Beispiel X

6-[1-(2-Aminoethyl)benzimidazol-5-yl]-3(2H)-pyridazinon

8,0 g (26,0 mMol) 6-[1-(2-Acetylaminoethyl)-benzimidazol-5-yl]-3(2H)-pyridazinon werden 4 Stunden lang in 80 ml 5n Natronlauge zum Rückfluß erhitzt. Nach dem Abkühlen wird mit konzentrierter Salzsäure auf pH 8-9 eingestellt, das ausgefallene Produkt abgesaugt und im Vakuumtrockenschrank getrocknet. Das so erhaltene Rohprodukt wird ohne weitere Reinigung weiter eingesetzt.
Ausbeute: 99 % der Theorie,
amorph

Beispiel XI

6-[4-(3-Aminopropylsulfonyl)phenyl]-4,5-dihydro-3(2H)-pyridazinon

30,0 g (70,7 mMol) 6-[4-(3-Phthalimidopropylsulfonyl)phenyl]-4,5-dihydro-3(2H)-pyridazinon werden in einem Gemisch aus 200 ml Toluol und 300 ml 40%iger wäßriger Methylaminlösung zwei Stunden lang bei Raumtemperatur heftig gerührt. Danach wird der ausgefallene Feststoff abgesaugt, nacheinander mit 100 ml Wasser, 50 ml Aceton und 50 ml Diethylether gewaschen und anschließend im Vakuumtrockenschrank über Phosphorpentoxid getrocknet.
Ausbeute: 20,2 g (97 % der Theorie),
Schmelzpunkt: ab 100° C (Zers.)

Beispiel XII

6-[4-(2-Aminoethoxy)-3-methyl-phenyl]-3(2H)-pyridazinon

Zu einer Mischung von 24,7 g (0,1 Mol) 6-[4-(2-Aminoethoxy)-3-methyl-phenyl]-4,5-dihydro-3(2H)-pyridazinon in 600 ml Eisessig und 9,8 g (0,1 Mol) konzentrierter Schwefelsäure werden unter Rühren langsam 19,2 g (0,12 Mol) Brom, gelöst in 20 ml Eisessig, getropft. Nach 1-stündigem Rühren bei 70 °C wird abgekühlt, noch 12 Stunden bei 20 °C gerührt, anschließend der gelbe Niederschlag abgesaugt und mit Aceton gewaschen. Zur Freisetzung der Base wird in 500 ml Wasser suspendiert und mit konzentriertem Ammoniak alkalisch gestellt. Das ausgefallene Produkt wird abgesaugt und getrocknet.
Ausbeute: 20,5 g (84 % der Theorie),
amorph
$R_f$-Wert: 0,30 (Kieselgel, Dichlormethan/Ethanol = 9:1)

| $C_{13}H_{15}N_3O_2$ (245,28) | | | |
|---|---|---|---|
| Ber.: | C 63,66 | H 6,16 | N 17,13 |
| Gef.: | 63,83 | 6,01 | 17,05 |

Beispiel 1

6-[4-[2-[3-(4-(2-Cyclopropylmethoxyethyl)phenoxy)-2-hydroxypropylamino]ethoxy]phenyl]-3(2H)-pyridazinon

Eine Lösung von 1,5 g (6 mMol) 1-[4-(2-Cyclopropylmethoxyethyl)phenoxy]-2,3-epoxypropan und 1,2 g (5 mMol) 6-[4-(2-Aminoethoxy)phenyl]-3(2H)-pyridazinon in 100 ml absolutem Ethanol wird sieben Stunden lang unter Rückfluß erhitzt. Das Gemisch wird anschließend bis zur Trockne eingedampft und das so angefallene Rohprodukt durch Chromatographie über 200 g Kieselgel (Dichlormethan/Methanol = 9:1) gereinigt.
Ausbeute: 950 mg (39,6 % der Theorie),
Schmelzpunkt: 134-135 °C

| $C_{27}H_{33}N_3O_5$ (479,58) | | | |
|---|---|---|---|
| Ber.: | C 67,62 | H 6,94 | N 8,76 |
| Gef.: | 67,46 | 6,94 | 8,58 |

Beispiel 2

6-[4-[2-[3-(4-(2-Isopropoxyethoxymethyl)phenoxy)-2-hydroxypropylamino]ethoxy]phenyl]-3(2H)-pyridazinon

Hergestellt analog Beispiel 1 aus 1-[4-(2-Isopropoxyethoxymethyl)phenoxy]-2,3-epoxypropan und 6-[4-(2-Aminoethoxy)phenyl]-3(2H)-pyridazinon.
Schmelzpunkt: 106-108 °C

| $C_{27}H_{35}N_3O_6$ (497,6) | | | |
|---|---|---|---|
| Ber.: | C 65,17 | H 7,09 | N 8,44 |
| Gef.: | 64,95 | 7,03 | 8,44 |

Beispiel 3

6-[4-[2-[3-(4-(2-Isobutoxy-ethyl)phenoxy)-2-hydroxypropylamino]ethoxy]-3-methyl-phenyl]-4,5-dihydro-3(2H)-pyridazinon

Hergestellt analog Beispiel 1 aus 1-[4-(2-Isobutoxy-ethyl)phenoxy]-2,3-epoxypropan und 6-[4-(2-Aminoethoxy)-3-methyl-phenyl]-4,5-dihydro-3(2H)-pyridazinon.
Ausbeute: 24 % der Theorie,
Schmelzpunkt: 119-120 ° C

| $C_{28}H_{39}N_3O_5$ (497,6) | | | |
|---|---|---|---|
| Ber.: | C 67,58 | H 7,90 | N 8,44 |
| Gef.: | 67,56 | 7,88 | 8,64 |

Beispiel 4

6-[4-[2-[3-(4-(2-Cyclopropylmethoxy-ethyl)phenoxy)-2-hydroxypropylamino]ethoxy]-3-methyl-phenyl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[4-(2-Aminoethoxy)-3-methyl-phenyl]-4,5-dihydro-3(2H)-pyridazinon.
Ausbeute: 20 % der Theorie,
Schmelzpunkt: 98-99 ° C

| $C_{28}H_{37}N_3O_5$ (495,6) | | | |
|---|---|---|---|
| Ber.: | C 67,86 | H 7,53 | N 8,48 |
| Gef.: | 67,83 | 7,61 | 8,55 |

Beispiel 5

6-[4-[2-[3-(4-(2-Isopropoxyethoxy-methyl)phenoxy)-2-hydroxypropylamino]ethoxy]-3-methyl-phenyl]-4,5-dihydro-3(2H)-pyridazinon

Hergestellt analog Beispiel 1 aus 1-[4-(2-Isopropoxyethoxymethyl)phenoxy]-2,3-epoxypropan und 6-[4-(2-Aminoethoxy)-3-methyl-phenyl]-4,5-dihydro-3(2H)-pyridazinon.
Ausbeute: 16 % der Theorie,
Schmelzpunkt: 95-96 ° C

| $C_{28}H_{39}N_3O_6$ (513,6) | | | |
|---|---|---|---|
| Ber.: | C 65,48 | H 7,65 | N 8,18 |
| Gef.: | 65,31 | 7,75 | 8,24 |

Beispiel 6

6-[4-[3-[3-(4-(2-Cyclopropylmethoxy-ethyl)phenoxy)-2-hydroxypropylamino]propoxy]-3-methyl-phenyl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[4-(3-Aminopropoxy)-3-methyl-phenyl]-4,5-dihydro-3(2H)-pyridazinon.
Ausbeute: 7 % der Theorie,
Schmelzpunkt: 142-145 °C

| $C_{29}H_{39}N_3O_5$ (509,6) | | | |
|---|---|---|---|
| Ber.: | C 68,35 | H 7,71 | N 8,24 |
| Gef.: | 68,13 | 7,62 | 8,10 |

Beispiel 7

6-[4-[3-[3-(4-(2-Cyclobutylmethoxy-ethyl)phenoxy)-2-hydroxypropylamino]propoxy]-3-methyl-phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[4-(3-Aminopropoxy)-3-methyl-phenyl]-4,5-dihydro-3(2H)-pyridazinon.
Ausbeute: 21 % der Theorie,
Schmelzpunkt: 149-152 °C

| $C_{30}H_{41}N_3O_5$ (523,7) | | | |
|---|---|---|---|
| Ber.: | C 68,81 | H 7,89 | N 8,02 |
| Gef.: | 68,72 | 7,71 | 7,73 |

Beispiel 8

6-[4-[3-[3-(4-(2-Isobutoxy-ethyl)phenoxy)-2-hydroxypropylamino]propoxy]-3-methyl-phenyl]-4.5-dihydro-3-(2H)-pyridazinon

Hergestellt analog Beispiel 1 aus 1-[4-(2-Isobutoxy-ethyl)phenoxy]-2,3-epoxypropan und 6-[4-(3-Aminopropoxy)-3-methyl-phenyl]-4,5-dihydro-3(2H)-pyridazinon.
Ausbeute: 18 % der Theorie,
Schmelzpunkt: 145-147 °C

| $C_{29}H_{41}N_3O_5$ (511,7) | | | |
|---|---|---|---|
| Ber.: | C 68,08 | H 8,08 | N 8,21 |
| Gef.: | 67,82 | 8,20 | 8,19 |

Beispiel 9

6-[4-[3-[3-(4-(2-Isopropoxyethoxy-methyl)phenoxy)-2-hydroxypropylamino]propoxy]-3-methyl-phenyl]-4,5-dihydro-3(2H)-pyridazinon

Hergestellt analog Beispiel 1 aus 1-[4-(2-Isopropoxyethoxymethyl)phenoxy]-2,3-epoxypropan und 6-[4-(3-Aminopropoxy)-3-methyl-phenyl]-4,5-dihydro-3(2H)-pyridazinon.
Ausbeute: 21 % der Theorie,
amorph

| $C_{29}H_{41}N_3O_6$ (527,7) | | | |
|---|---|---|---|
| Ber.: | C 66,01 | H 7,83 | N 7,96 |
| Gef.: | 65,88 | 7,89 | 7,78 |

$^1$H-NMR-Spektrum ($CDCl_3/CD_3OD$):
$\delta$ = 1,18 (d,6H); 2,13 (m,2H); 2,21 (s,3H); 2,56 (t,2H); 2,97 (m,6H); 3,60 (m,5H); 3,90-4,90 (m,5H); 4,50 (s,2H) 6,88 (m,3H); 7,27 (m,2H); 7,50 (m,2H) ppm.

## Beispiel 10

6-[4-[3-[3-(4-(2-Cyclobutylmethoxy-ethyl)phenoxy)-2-hydroxypropylamino]propoxy]-3-chlor-phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[4-(3-Aminopropoxy)-3-chlor-phenyl]-4,5-dihydro-3(2H)-pyridazinon.
Ausbeute: 25 % der Theorie,
Schmelzpunkt: 120-122 °C

| $C_{29}H_{38}ClN_3O_5$ (544,1) | | | | |
|---|---|---|---|---|
| Ber.: | C 64,02 | H 7,04 | N 7,72 | Cl 6,52 |
| Gef.: | 63,88 | 6,87 | 7,82 | 6,82 |

## Beispiel 11

6-[4-[3-[3-(4-(2-Cyclopropylmethoxy-ethyl)phenoxy)-2-hydroxypropylamino]propoxy]-3-chlor-phenyl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[4-(3-Aminopropoxy)-3-chlor-phenyl]-4,5-dihydro-3(2H)-pyridazinon.
Ausbeute: 33 % der Theorie,
Schmelzpunkt: 118-120 °C

| $C_{28}H_{36}ClN_3O_5$ (530,1) | | | | |
|---|---|---|---|---|
| Ber.: | C 63,44 | H 6,85 | N 7,93 | Cl 6,69 |
| Gef.: | 63,34 | 6,83 | 7,95 | 6,91 |

Beispiel 12

6-[4-[3-[3-(4-(2-Isopropoxyethoxy-methyl)phenoxy)-2-hydroxypropylamino]propoxy]-3-chlor-phenyl]-4,5-dihydro-3(2H)-pyridazinon

Hergestellt analog Beispiel 1 aus 1-[4-(2-Isopropoxyethoxymethyl)phenoxy]-2,3-epoxypropan und 6-[4-(3-Aminopropoxy)3-chlor-phenyl]-4,5-dihydro-3(2H)-pyridazinon.
Ausbeute: 15 % der Theorie,
Schmelzpunkt: 187-189 ° C

| $C_{28}H_{38}ClN_3O_6$ x HCL (584,6) | | | | |
|---|---|---|---|---|
| Ber.: | C 57,52 | H 6,72 | N 7,19 | Cl 12,13 |
| Gef.: | 57,44 | 6,59 | 7,14 | 12,16 |

Beispiel 13

6-[4-[2-[3-(4-(2-Cyclobutylmethoxy-ethyl)phenoxy)-2-hydroxyPropylamino]ethoxy]-3-chlor-phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[4-(2-Aminoethoxy)-3-chlor-phenyl]-4,5-dihydro-3(2H)-pyridazinon.
Ausbeute: 27 % der Theorie,
Schmelzpunkt: 128-130 ° C

| $C_{28}H_{36}ClN_3O_5$ (530,1) | | | | |
|---|---|---|---|---|
| Ber.: | C 63,44 | H 6,85 | N 7,93 | Cl 6,69 |
| Gef.: | 63,52 | 6,71 | 8,02 | 6,89 |

Beispiel 14

6-[4-[2-[3-(4-(2-Isobutoxy-ethyl)phenoxy)-2-hydroxypropylamino]ethoxy]-3-chlor-phenyl]-4,5-dihydro-3(2H)-pyridazinon

Hergestellt analog Beispiel 1 aus 1-[4-(2-Isobutoxy-ethyl)phenoxy]-2,3-epoxypropan und 6-[4-[2-Aminoethoxy)-3-chlor-phenyl]-4,5-dihydro-3(2H)-pyridazinon.
Ausbeute: 33 % der Theorie,
Schmelzpunkt: 127-129 ° C

| $C_{27}H_{36}ClN_3O_5$ (518,1) | | | | |
|---|---|---|---|---|
| Ber.: | C 62,59 | H 7,00 | N 8,11 | Cl 6,84 |
| Gef.: | 62,46 | 7,02 | 8,14 | 7,06 |

Beispiel 15

6-[4-[2-[3-(4-(2-Isopropoxyethoxy-methyl)phenoxy)-2-hydroxypropylamino]ethoxy]-3-chlor-phenyl]-4,5-dihydro-3(2H)-pyridazinon

Hergestellt analog Beispiel 1 aus 1-[4-(2-Isopropoxyethoxymethyl)phenoxy]-2,3-epoxypropan und 6-[4-(2-Aminoethoxy)-3-chlor-phenyl]-4,5-dihydro-3(2H)-pyridazinon.
Ausbeute: 19 % der Theorie,
Schmelzpunkt: 96-99 °C

| $C_{27}H_{36}ClN_3O_6$ (534,1) | | | | |
|---|---|---|---|---|
| Ber.:<br>Gef.: | C 60,71<br>60,59 | H 6,79<br>6,90 | N 7,87<br>8,10 | Cl 6,64<br>6,45 |

Beispiel 16

6-[4-[2-[3-(Naphthyl-1-oxy)-2-hydroxypropylamino]ethoxy]phenyl]-4,5-dihydro-5-methyl-3(2H)-pyridazinon

Hergestellt analog Beispiel 1 aus 1-(Naphthyl-1-oxy)-2,3-epoxypropan und 6-[4-(2-Aminoethoxy)phenyl]-4,5-dihydro-5-methyl-3(2H)-pyridazinon.
Ausbeute: 34 % der Theorie,
amorph

| $C_{26}H_{29}N_3O_4$ (447,5) | | | |
|---|---|---|---|
| Ber.:<br>Gef.: | C 69,78<br>69,54 | H 6,53<br>6,44 | N 9,39<br>9,35 |

$^1$H-NMR-Spektrum ($CDCl_3/CD_3OD$):
$\delta$ = 1,22 (d,3H); 2,42 (dd,1H); 2,71 (dd,1H); 2,9-3,2 (m,4H); 3,3-3,45 (m,1H); 4,1-4,4 (m,6H); 6,8-7,0 (m,3H); 7,3-7,6 (m,4H) 7,65-7,85 (m,3H); 8,25 (m,1H) ppm.

Beispiel 17

6-[4-[2-[3-(2-Chlor-5-methyl-phenoxy)-2-hydroxypropylamino]ethoxy]phenyl]-4,5-dihydro-5-methyl-3(2H)-pyridazinon

Hergestellt analog Beispiel 1 aus 1-(2-Chlor-5-methylphenoxy)-2,3-epoxypropan und 6-[4-(2-Aminoethoxy)phenyl]-4,5-dihydro-5-methyl-3(2H)-pyridazinon.
Ausbeute: 37 % der Theorie,
amorph

| $C_{23}H_{28}ClN_3O_4$ (445,9) | | | | |
|---|---|---|---|---|
| Ber.:<br>Gef.: | C 61,95<br>61,84 | H 6,33<br>6,39 | N 9,42<br>9,45 | Cl 7,95<br>7,96 |

$^1$H-NMR-Spektrum ($CDCl_3/CD_3OD$):
$\delta$ = 1,22 (d,3H); 2,31 (s,3H); 2,40 (dd,1H); 2,70 (dd,1H); 2,85-3,20 (m,4H); 3,86 (m,1H); 3,95-4,20 (m,5H); 6,75 (m,2H); 6,95 (d,2H); 7,20 (d,1H); 7,70 (d,2H) ppm.

## Beispiel 18

6-[4-[2-[3-(2-Cyano-phenoxy)-2-hydroxypropylamino]ethoxy]phenyl]-4,5-dihydro-5-methyl-3(2H)-pyridazinon

Hergestellt analog Beispiel 1 aus 1-(2-Cyano-phenoxy)-2,3-epoxypropan und 6-[4-(2-Aminoethoxy)-phenyl]-4,5-dihydro-5-methyl-3(2H)-pyridazinon.
Ausbeute: 23 % der Theorie,
amorph

| $C_{23}H_{26}N_4O_4$ (422,5) | | | |
|---|---|---|---|
| Ber.: | C 65,39 | H 6,20 | N 13,26 |
| Gef.: | 65,28 | 6,32 | 13,13 |

$^1$H-NMR-Spektrum ($CDCl_3/CD_3OD$):
$\delta$ = 1,22 (d,3H); 2,42 (dd,1H); 2,72 (dd,1H); 2,85-3,15 (m,4H); 3,37 (m,1H); 4,05-4,25 (m,5H); 6,90-7,10 (m,4H); 7,90-7,80 (m,4H) ppm.

## Beispiel 19

6-[4-[2-[3-(2-Allyloxy-phenoxy)-2-hydroxypropylamino]ethoxy]phenyl]-4,5-dihydro-5-methyl-3(2H)-pyridazinon

Hergestellt analog Beispiel 1 aus 1-(2-Allyloxy-phenoxy)-2,3-epoxypropan und 6-[4-(2-Aminoethoxy)-phenyl]-4,5-dihydro-5-methyl-3(2H)-pyridazinon.
Ausbeute: 39 % der Theorie,
amorph

| $C_{25}H_{31}N_3O_5$ (453,5) | | | |
|---|---|---|---|
| Ber.: | C 66,21 | H 6,89 | N 9,26 |
| Gef.: | 65,92 | 7,00 | 9,17 |

$^1$H-NMR-Spektrum ($CDCl_3/CD_3OD$):
$\delta$ = 1,22 (d,3H); 2,42 (dd,1H); 2,72 (dd,1H); 2,90 (m,2H); 3,10 (t,2H); 3,38 (m,1H); 3,95-4,20 (m,5H); 4,60 (m,2H) 5,25-5,50 (m,2H); 6,10 (m,1H); 6,90-7,00 (m,6H); 7,70 (d,2H) ppm.

## Beispiel 20

6-[4-[2-[3-(3,4-Dihydro-carbostyril-5-oxy)-2-hydroxypropylamino]ethoxy]phenyl]-4,5-dihydro-5-methyl-3(2H)-pyridazinon

Hergestellt analog Beispiel 1 aus 1-(3,4-Dihydro-carbosty ril-5-oxy)-2,3-epoxypropan und 6-[4-(2-Aminoethoxy)phenyl]4,5-dihydro-5-methyl-3(2H)-pyridazinon.
Ausbeute: 32 % der Theorie,

amorph

| $C_{25}H_{30}N_4O_5$ (466,5) | | | |
|---|---|---|---|
| Ber.: | C 64,36 | H 6,48 | N 12,01 |
| Gef.: | 64,12 | 6,61 | 11,85 |

$^1$H-NMR-Spektrum ($CDCl_3/CD_3OD$):
$\delta$ = 1,23 (d,3H); 2,38-3,40 (m,11H); 3,95-4,20 (m,5H); 6,48 (d,1H); 6,60 (d,1H); 6,93 (d,2H); 7,11 (t,1H); 7,70 (d,2H) ppm.

Beispiel 21

6-[4-[2-[3-(4-(2-Cyclobutylmethoxy-ethyl)phenoxy)-2-hydroxypropylamino]ethoxy]phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[4-(2-Aminoethoxy)phenyl]-4,5-dihydro-3(2H)-pyridazinon.
Ausbeute: 27 % der Theorie,
Schmelzpunkt: 116-117° C

| $C_{28}H_{37}N_3O_5$ (495,6) | | | |
|---|---|---|---|
| Ber.: | C 67,86 | H 7,53 | N 8,48 |
| Gef.: | 67,69 | 7,51 | 8,44 |

Beispiel 22

6-[4-[2-[3-(4-((2-Isopropoxyethoxy)methyl)phenoxy)-2-hydroxypropyl-amino]ethoxy]phenyl]-4,5-dihydro-3-(2H)-pyridazinon

Hergestellt analog Beispiel 1 aus 1-[4-((2-Isopropoxyethoxy) methyl)phenoxy]-2,3-epoxypropan und 6-[4-(2-Aminoethoxy)phenyl]-4,5-dihydro-3(2H)-pyridazinon.
Ausbeute: 25 % der Theorie,
Schmelzpunkt: 102-105° C

| $C_{27}H_{37}N_3O_6$ (499,6) | | | |
|---|---|---|---|
| Ber.: | C 64,91 | H 7,46 | N 8,41 |
| Gef.: | 64,89 | 7,64 | 8,46 |

Beispiel 23

6-[4-[2-[3-(4-(2-Cyclopropylmethoxy-ethyl)phenoxy)-2-hydroxypropylamino]ethoxy]phenyl]-4,5-dihydro-3-(2H)-pyridazinon

Hergestellt analog Beispiel 1 aus 1-[4-(2-Cyclopropylmethoxy-ethyl)phenoxy]-2,3-epoxypropan und 6-[4-(2-Aminoethoxy)phenyl]-4,5-dihydro-3(2H)-pyridazinon.
Ausbeute: 37 % der Theorie,
Schmelzpunkt: 121-122 °C

| $C_{27}H_{35}N_3O_5$ (481,6) | | | |
|---|---|---|---|
| Ber.: | C 67,34 | H 7,33 | N 8,73 |
| Gef.: | 67,13 | 7,34 | 8,59 |

Beispiel 24

6-[4-[3-[3-(4-(2-Cyclopropylmethoxy-ethyl)phenoxy)-2-hydroxypropylamino]propoxy]phenyl]-4,5-dihydro-3-(2H)-pyridazinon

Hergestellt analog Beispiel 1 aus 1-[4-(2-Cyclopropylmethoxy-ethyl)phenoxy]-2,3-epoxypropan und 6-[4-(3-Aminopropoxy)phenyl]-4,5-dihydro-3(2H)-pyridazinon.
Ausbeute: 19 % der Theorie,
Schmelzpunkt: ab 113 °C (Sintern)

| $C_{28}H_{37}N_3O_5$ (495,6) | | | |
|---|---|---|---|
| Ber.: | C 67,86 | H 7,53 | N 8,48 |
| Gef.: | 67,85 | 7,44 | 8,29 |

Beispiel 25

6-[3-[2-[3-(Naphthyl-1-oxy)-2-hydroxypropylamino]ethoxy]phenyl]-4,5-dihydro-5-methyl-3(2H)-pyridazinon

Hergestellt analog Beispiel 1 aus 1-(Naphthyl-1-oxy)-2,3-epoxypropan und 6-[3-(2-Aminoethoxy)phenyl]-4,5-dihydro-5-methyl-3(2H)-pyridazinon.
Ausbeute: 36 % der Theorie,
amorph

| $C_{26}H_{29}N_3O_4$ (447,5) | | | |
|---|---|---|---|
| Ber.: | C 69,78 | H 6,53 | N 9,39 |
| Gef.: | 69,58 | 6,55 | 9,33 |

$^1$H-NMR-Spektrum ($CDCl_3/CD_3OD$):
$\delta$ = 1,22 (d,3H); 2,42 (dd,1H); 2,70 (dd,1H); 2,90-3,40 (m,5H); 4,00-4,35 (m,5H); 6,85 (dd,1H); 6,98 (m,1H); 7,30-7,55 (m,7H); 7,80 (dd,1H); 8,35 (dd,1H) ppm.

Beispiel 26

6-[3-[2-[3-(2-Cyano-phenoxy)-2-hydroxypropylamino]ethoxy]phenyl]-4,5-dihydro-5-methyl-3(2H)-pyridazinon

Hergestellt analog Beispiel 1 aus 1-(2-Cyano-phenoxy)-2,3-epoxypropan und 6-[3-(2-Aminoethoxy)-phenyl]-4,5-dihydro-5-methyl-3(2H)-pyridazinon.
Ausbeute: 27 % der Theorie,
amorph

| $C_{23}H_{26}N_4O_4$ (422,5) | | | |
|---|---|---|---|
| Ber.:<br>Gef.: | C 65,39<br>65,12 | H 6,20<br>6,11 | N 13,26<br>13,09 |

$^1$H-NMR-Spektrum ($CDCl_3/CD_3OD$):
δ = 1,24 (d,3H); 2,40-3,40 (m,7H); 4,05-4,55 (m,5H); 7,00 (m,3H); 7,25-7,40 (m,3H); 7,45-7,60 (m,2H) ppm.

Beispiel 27

6-[3-[2-[3-(3,4-Dihydro-carbostyril-5-oxy)-2-hydroxypropylamino]ethoxy]phenyl]-4,5-dihydro-5-methyl-3(2H)-pyridazinon

Hergestellt analog Beispiel 1 aus 1-(3,4-Dihydro-carbostyril-5-oxy)-2,3-epoxypropan und 6-[3-(2-Aminoethoxy)phenyl]-4,5-dihydro-5-methyl-3(2H)-pyridazinon.
Ausbeute: 43 % der Theorie,
amorph

| $C_{25}H_{30}N_4O_5$ (466,5) | | | |
|---|---|---|---|
| Ber.:<br>Gef.: | C 64,36<br>64,35 | H 6,48<br>6,57 | N 12,01<br>11,78 |

$^1$H-NMR-Spektrum ($CDCl_3/CD_3OD$):
δ = 1,22 (d,3H); 2,40-3,40 (m,10H); 3,95-4,20 (m,5H); 6,50 (d,1H); 6,60 (d,1H); 6,98 (m,1H); 7,11 (t,1H); 7,30-7,42 (m,3H) ppm.

Beispiel 28

6-[3-[2-[3-(2-Chlor-5-methyl-phenoxy)-2-hydroxypropylamino]ethoxy]phenyl]-4,5-dihydro-5-methyl-3(2H)-pyridazinon

Hergestellt analog Beispiel 1 aus 1-(2-Chlor-5-methyl-phen oxy)-2,3-epoxypropan und 6-[3-(2-Aminoethoxy)phenyl]-4,5-dihydro-5-methyl-3(2H)-pyridazinon.
Ausbeute: 22 % der Theorie,
amorph

| $C_{23}H_{28}ClN_3O_4$ (445,9) | | | | |
|---|---|---|---|---|
| Ber.:<br>Gef.: | C 61,95<br>61,78 | H 6,33<br>6,21 | N 9,42<br>9,33 | Cl 7,95<br>8,03 |

$^1$H-NMR-Spektrum ($CDCl_3/CD_3OD$):
δ = 1,22 (d,3H); 2,31 (s,3H); 2,35-3,40 (m,7H); 4,00-4,25 (m,5H); 6,75 (m,2H); 6,99 (m,1H); 7,20 (d,1H); 7,33 (m,3H) ppm.

Beispiel 29

6-[3-[2-[3-(2-Allyloxy-phenoxy)-2-hydroxypropylamino]ethoxy]phenyl]-4,5-dihydro-5-methyl-3(2H)-pyridazinon

Hergestellt analog Beispiel 1 aus 1-(2-Allyloxy-phenoxy)-2,3-epoxypropan und 6-[3-(2-Aminoethoxy)-phenyl]-4,5-dihydro-5-methyl-3(2H)-pyridazinon.
Ausbeute: 33 % der Theorie,
amorph

| $C_{25}H_{31}N_3O_5$ (453,5) | | | |
|---|---|---|---|
| Ber.: | C 66,21 | H 6,89 | N 9,26 |
| Gef.: | 66,05 | 6,98 | 9,11 |

$^1$H-NMR-Spektrum ($CDCl_3/CD_3OD$): $\delta$ = 1,22 (d,3H); 2,35-3,40 (m,7H); 3,95-4,20 (m,5H); 4,56 (m,2H); 5,20-5,50 (m,2H); 5,95-6,20 (m,1H); 6,80-7,00 (m,5H); 7,20-7,40 (m,3H) ppm.

Beispiel 30

6-[3-[2-[3-(4-(2-Cyclopropylmethoxy-ethyl)phenoxy)-2-hydroxypropylamino]ethoxy]phenyl]-4,5-dihydro-3-(2H)-pyridazinon

Hergestellt analog Beispiel I aus 1-[4-(2-Cyclopropylmethoxy-ethyl)phenoxy]-2,3-epoxypropan und 6-[3-(2-Aminoethoxy)phenyl]-4,5-dihydro-3(2H)-pyridazinon.
Ausbeute: 25 % der Theorie,
Schmelzpunkt: 102-104 °C

| $C_{27}H_{35}N_3O_5$ (481,6) | | | |
|---|---|---|---|
| Ber.: | C 67,34 | H 7,33 | N 8,73 |
| Gef.: | 67,15 | 7,21 | 8,77 |

Beispiel 31

6-[3-[2-[3-(4-(2-Isobutoxy-ethyl)phenoxy)-2-hydroxy-propylamino]ethoxy]phenyl]-4,5-dihydro-3(2H)-pyridazinon

Hergestellt analog Beispiel 1 aus 1-[4-(2-Isobutoxy-ethyl)phenoxy]-2,3-epoxypropan und 6-[3-(2-Aminoethoxy)phenyl]-4,5-dihydro-3(2H)-pyridazinon.
Ausbeute: 14 % der Theorie,
Schmelzpunkt: 109-110 °C

| $C_{27}H_{37}N_3O_5$ (483,6) | | | |
|---|---|---|---|
| Ber.: | C 67,06 | H 7,71 | N 8,69 |
| Gef.: | 66,86 | 7,54 | 8,64 |

Beispiel 32

6-[3-[2-[3-(4-(2-Cyclobutylmethoxy-ethyl)phenoxy)-2-hydroxypropylamino]ethoxy]phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[3-(2-Aminoethoxy)phenyl]-4,5-dihydro-3(2H)-pyridazinon.
Ausbeute: 19 % der Theorie,
Schmelzpunkt: 102-103 °C

| $C_{28}H_{37}N_3O_5$ (495,6) | | | |
|---|---|---|---|
| Ber.: | C 67,86 | H 7,53 | N 8,48 |
| Gef.: | 67,60 | 7,38 | 8,32 |

Beispiel 33

6-[3-[3-[3-(4-(2-Cyclopropylmethoxy-ethyl)phenoxy)-2-hydroxypropylamino]propoxy]phenyl]-4,5-dihydro-3-(2H)-pyridazinon

Hergestellt analog Beispiel 1 aus 1-[4-(2-Cyclopropylmethoxyethyl)phenoxy]-2,3-epoxypropan und 6-[3-(3-Aminopropoxy)phenyl]-4,5-dihydro-3(2H)-pyridazinon.
Ausbeute: 12 % der Theorie,
Schmelzpunkt: 182-183 °C

| $C_{28}H_{37}N_3O_5$ (495,6) | | | |
|---|---|---|---|
| Ber.: | C 67,86 | H 7,53 | N 8,48 |
| Gef.: | 67,57 | 7,49 | 8,40 |

Beispiel 34

6-[2-[4-[3-(2-Cyano-phenoxy)-2-hydroxypropylamino]butylamino]benzoxazol-5-yl]-4,5-dihydro-5-methyl-3-(2H)-pyridazinon

Hergestellt analog Beispiel 1 aus 1-(2-Cyano-phenoxy)-2,3-epoxypropan und 6-[2-(4-Aminobutylamino)-benzoxazol-5-yl]-4,5-dihydro-5-methyl-3(2H)-pyridazinon.
Ausbeute: 42 % der Theorie,
amorph

| $C_{26}H_{30}N_6O_4$ (490,6) | | | |
|---|---|---|---|
| Ber.: | C 63,65 | H 6,16 | N 17,13 |
| Gef.: | 63,76 | 6,18 | 16,98 |

$^1$H-NMR-Spektrum ($CDCl_3/CD_3OD$):
$\delta$ = 1,25 (d,3H); 1,60-1,90 (m,4H); 2,45 (dd,1H); 2,65-3,10 (m,7H); 3,30-3,60 (m,3H); 4,05-4,30 (m,3H); 6,45-7,30 (m,3H); 7,40-7,60 (m,3H); 7,76 (s,1H) ppm.

Beispiel 35

6-[2-[4-[3-(Naphthyl-1-oxy)-2-hydroxypropylamino]butylamino]benzoxazol-5-yl]-4,5-dihydro-5-methyl-3(2H)-pyridazinon

Hergestellt analog Beispiel 1 aus 1-(Naphthyl-1-oxy)-2,3-epoxypropan und 6-[2-(4-Aminobutylamino)-benzoxazol-5-yl]-4,5-dihydro-5-methyl-3(2H)-pyridazinon.
Ausbeute: 41 % der Theorie,
amorph

| $C_{29}H_{33}N_5O_4$ (515,6) | | | |
|---|---|---|---|
| Ber.: | C 67,55 | H 6,45 | N 13,58 |
| Gef.: | 67,42 | 6,22 | 13,33 |

δ = 1,22 (d,3H); 1,62-1,40 (m,4H); 2,40 (dd,1H); 2,60-3,10 (m,5H); 3,31 (m,1H); 3,49 (t,2H); 4,12-4,40 (m,3H); 6,82 (dd,1H); 7,17-7,53 (m,6H); 7,70-7,84 (m,2H); 8,23 (dd,1H) ppm.

Beispiel 36

6-[2-[3-[3-(2-cyano-phenoxy)-2-hydroxypropylamino]propylamino]benzoxazol-5-yl]-4,5-dihydro-5-methyl-3-(2H)-pyridazinon

Hergestellt analog Beispiel 1 aus 1-(2-Cyano-phenoxy)-2,3-epoxypropan und 6-[2-(3-Aminopropylamino)benzoxazol-5-yl]-4,5-dihydro-5-methyl-3(2H)-pyridazinon.
Ausbeute: 47 % der Theorie,
amorph

| $C_{25}H_{28}N_6O_4$ (476,5) | | | |
|---|---|---|---|
| Ber.: | C 63,01 | H 5,92 | N 17,64 |
| Gef.: | 62,89 | 5,81 | 17,49 |

$^1$H-NMR-Spektrum ($CDCl_3$/$CD_3OD$):
δ = 1,24 (d,3H); 1,84-2,00 (m,2H); 2,45 (dd,1H); 2,65-3,05 (m,7H); 3,38 (m,1H); 3,66 (t,2H); 4,10-4,30 (m,3H); 7,00 (m,2H); 7,20 (d,1H); 7,38-7,60 (m,3H); 7,88 (s,1H) ppm.

Beispiel 37

6-[2-[3-[3-(Naphthyl-1-oxy)-2-hydroxypropylamino]propylamino]benzoxazol-5-yl]-4,5-dihydro-5-methyl-3(2H)-pyridazinon

Hergestellt analog Beispiel 1 aus 1-(Naphthyl-1-oxy)-2,3-epoxypropan und 6-[2-(3-Aminopropylamino)-benzoxazol-5-yl]-4,5-dihydro-5-methyl-3(2H)-pyridazinon.
Ausbeute: 22 % der Theorie,
amorph

| $C_{28}H_{31}N_5O_4$ (501,6) | | | |
|---|---|---|---|
| Ber.: | C 67,05 | H 6,23 | N 13,96 |
| Gef.: | 66,91 | 6,11 | 14,12 |

$^1$H-NMR-Spektrum ($CDCl_3$/$CD_3OD$):
δ = 1,24 (d,3H); 1,92 (m,2H); 2,40 (dd,1H); 2,62-3,05 (m,5H); 3,36 (m,1H); 3,55 (t,2H); 4,10-4,40 (m,3H); 6,85 (dd,1H); 7,18-7,53 (m,6H); 7,80 (m,2H); 8,20 (dd,1H) ppm.

Beispiel 38

6-[2-[2-[3-(2-Cyano-phenoxy)-2-hydroxypropylamino]ethyl]benzimidazol-5-yl]-4,5-dihydro-3(2H)-pyridazinon

Hergestellt analog Beispiel 1 aus 1-(2-Cyano-phenoxy)-2,3-epoxypropan und 6-[2-(2-Aminoethyl)-benzimidazol-5-yl]-4,5-dihydro-3(2H)-pyridazinon.
Ausbeute: 18 % der Theorie,
Schmelzpunkt: 183-184 °C

| $C_{23}H_{24}N_6O_3$ (432,5) | | | |
|---|---|---|---|
| Ber.: | C 63,88 | H 5,59 | N 19,43 |
| Gef.: | 63,70 | 5,45 | 19,40 |

Beispiel 39

6-[2-[2-[3-(Naphthyl-1-oxy)-2-hydroxypropylamino]ethyl]benzimidazol-5-yl]-4,5-dihydro-3(2H)-pyridazinon

Hergestellt analog Beispiel 1 aus 1-(Naphthyl-1-oxy)-2,3- epoxypropan und 6-[2-(2-Aminoethyl)-benzimidazol-5-yl]-4,5-dihydro-3(2H)-pyridazinon.
Ausbeute: 22 % der Theorie,
Schmelzpunkt: 203-204 °C

| $C_{26}H_{27}N_5O_3$ (457,5) | | | |
|---|---|---|---|
| Ber.: | C 68,25 | H 5,95 | N 15,31 |
| Gef.: | 68,10 | 5,88 | 15,48 |

Beispiel 40

6-[2-[2-[3-(2-Chlor-5-methyl-phenoxy)-2-hydroxypropylamino]ethyl]benzimidazol-5-yl]-4,5-dihydro-3(2H)-pyridazinon

Hergestellt analog Beispiel 1 aus 1-(2-Chlor-5-methyl-phenoxy)-2,3-epoxypropan und 6-[2-(2-Aminoethyl)benzimidazol-5-yl]-4,5-dihydro-3(2H)-pyridazinon.
Ausbeute: 20 % der Theorie,
Schmelzpunkt: 182-183 °C

| $C_{23}H_{26}ClN_5O_3$ (455,9) | | | | |
|---|---|---|---|---|
| Ber.: | C 60,59 | H 5,75 | N 15,36 | Cl 7,78 |
| Gef.: | 60,49 | 5,75 | 15,62 | 8,03 |

Beispiel 41

6-[2-[2-[3-(2-Allyloxy-phenoxy)-2-hydroxypropylamino]ethyl]benzimidazol-5-yl]-4,5-dihydro-3(2H)-pyridazinon

Hergestellt analog Beispiel 1 aus 1-(2-Allyloxy-phenoxy)-2,3-epoxypropan und 6-[2-(2-Aminoethyl)-benzimidazol-5-yl]-4,5-dihydro-3(2H)-pyridazinon.
Ausbeute: 9 % der Theorie,
Schmelzpunkt: 172-173 ° C

| $C_{25}H_{29}N_5O_4$ (463,5) | | | |
|---|---|---|---|
| Ber.: | C 64,78 | H 6,31 | N 15,11 |
| Gef.: | 64,72 | 6,38 | 15,20 |

Beispiel 42

6-[2-[2-[3-(4-(2-Isobutoxy-ethyl)phenoxy)-2-hydroxypropylamino]ethyl]benzimidazol-5-yl]-4,5-dihydro-3(2H)-pyridazinon

Hergestellt analog Beispiel 1 aus 1-(4-[2-Isobutoxy-ethyl)phenoxy]-2,3-epoxypropan und 6-[2-(2-Aminoethyl)benzimidazol-5-yl]-4,5-dihydro-3(2H)-pyridazinon.
Ausbeute: 9 % der Theorie,
amorph, $R_f$-Wert: 0,47 (Kieselgel, Dichlormethan/Methanol/Ammoniak = 80:20:0,5)

| $C_{28}H_{37}N_5O_4$ (507,6) | | | |
|---|---|---|---|
| Ber.: | C 66,25 | H 7,35 | N 13,80 |
| Gef.: | 65,92 | 7,19 | 13,99 |

Beispiel 43

6-[2-[2-[3-(Naphthyl-1-oxy)-2-hydroxypropylamino]ethyl]benzimidazol-5-yl]-4,5-dihydro-5-methyl-3(2H)-pyridazinon

Hergestellt analog Beispiel 1 aus 1-(Naphthyl-1-oxy)-2,3-epoxypropan und 6-[2-(2-Aminoethyl)-benzimidazol-5-yl]-4,5-dihydro-5-methyl-3(2H)-pyridazinon.
Ausbeute: 30 % der Theorie,
amorph

| $C_{27}H_{29}N_5O_3$ (471,6) | | | |
|---|---|---|---|
| Ber.: | C 68,77 | H 6,20 | N 14,85 |
| Gef.: | 68,57 | 6,18 | 14,64 |

$^1$H-NMR-Spektrum ($d_6$-DMSO/$CD_3OD$):
δ = 1,13 (d,3H); 2,28 (dd,1H); 2,65-3,20 (m,7H); 3,47 (m,1H); 4,05-4,20 (m,3H); 6,91 (dd,1H); 7,35-7,56 (m,5H); 7,70 (dd,1H); 7,80-7,90 (m,2H); 8,27 (m,1H) ppm.

Beispiel 44

6-[2-[2-[3-(4-Aminocarbonylmethyl-phenoxy)-2-hydroxypropylamino]-ethyl]benzimidazol-5-yl]-4,5-dihydro-5-methyl-3(2H)-pyridazinon

Hergestellt analog Beispiel 1 aus 1-(4-Aminocarbonylmethylphenoxy)-2,3-epoxypropan und 6-[2-(2-Aminoethyl)benzimidazol-5-yl]-4,5-dihydro-5-methyl-3(2H)-pyridazinon.
Ausbeute: 12 % der Theorie,
amorph

| $C_{25}H_{30}N_6O_4$ (478,6) | | | |
|---|---|---|---|
| Ber.: | C 62,75 | H 6,32 | N 17,56 |
| Gef.: | 62,73 | 6,23 | 17,33 |

$^1$H-NMR-Spektrum ($d_6$-DMSO/$CD_3OD$):
δ = 1,14 (d,3H); 2,28 (dd,1H); 2,60-2,83 (m,3H); 3,02 (m,4H); 3,30 (m,2H); 3,48 (m,1H); 3,80-4,00 (m,3H); 6,84 (d,2H); 7,18 (d,2H); 7,50 (d,1H); 7,69 (dd,1H); 7,89 (d,1H) ppm.

Beispiel 45

6-[2-[2-[3-(3,4-Dihydro-carbostyril-5-oxy)-2-hydroxypropylamino]-ethyl]benzimidazol-5-yl]-4,5-dihydro-5-methyl-3(2H)-pyridazinon

Hergestellt analog Beispiel 1 aus 1-(3,4-Dihydro-carbostyril- 5-oxy)-2,3-epoxypropan und 6-[2-(2-Aminoethyl)benzimidazol-5-yl]-4,5-dihydro-5-methyl-3(2H)-pyridazinon.
Ausbeute: 19 % der Theorie,
Schmelzpunkt: ab 130 °C (Sintern)

| $C_{26}H_{30}N_6O_4$ (490,6) | | | |
|---|---|---|---|
| Ber.: | C 63,66 | H 6,16 | N 17,13 |
| Gef.: | 63,49 | 6,29 | 16,82 |

Beispiel 46

6-[2-[2-[3-(2-Allyloxy-phenoxy)-2-hydroxypropylamino]ethyl]benzimidazol-5-yl]-4,5-dihydro-5-methyl-3(2H)-pyridazinon

Hergestellt analog Beispiel 1 aus 1-(2-Allyloxy-phenoxy)2,3-epoxypropan und 6-[2-(2-Aminoethyl)-benzimidazol-5-yl]-4,5-dihydro-5-methyl-3(2H)-pyridazinon.
Ausbeute: 39 % der Theorie,
amorph

| $C_{26}H_{31}N_5O_4$ (477,6) | | | |
|---|---|---|---|
| Ber.:<br>Gef.: | C 65,39<br>65,21 | H 6,54<br>6,66 | N 14,66<br>14,66 |

$^1$H-NMR-Spektrum ($CDCl_3/CD_3OD$):
$\delta$ = 1,27 (d,3H); 2,46 (dd,1H); 2,70-3,20 (m,8H); 3,48 (m,1H); 4,00 (m,2H); 4,18 (m,2H); 4,58 (dt,2H); 5,25-5,47 (m,2H); 5,95-6,16 (m,1H); 6,92 (s,4H); 7,50 (m,1H); 7,71 (dd,1H); 7,89 (d,1H) ppm.

Beispiel 47

6-[2-[2-[3-(4-(2-Cyclobutylmethoxy-ethyl)phenoxy)-2-hydroxypropylamino]ethyl]benzimidazol-5-yl]-4,5-dihydro-5-methyl-3(2H)-pyridazinon-dihydrochlorid

Hergestellt analog Beispiel 1 aus 1-[4-(2-Cyclobutylmethoxyethyl)phenoxy]-2,3-epoxypropan und 6-[2-(2-Aminoethyl)benzimidazol-5-yl]-4,5-dihydro-5-methyl-3(2H)-pyridazinon.
Ausbeute: 15 % der Theorie,
Schmelzpunkt: 194-196 °C

| $C_{30}H_{39}N_5O_4$ x 2 HCl (606,6) | | | | |
|---|---|---|---|---|
| Ber.:<br>Gef.: | C 59,40<br>59,23 | H 6,81<br>6,78 | N 11,55<br>11,62 | Cl 11,69<br>11,79 |

Beispiel 48

6-[2-[2-[3-(4-(2-Isobutoxy-ethyl)phenoxy)-2-hydroxypropylamino]ethyl]benzimidazol-5-yl]-4,5-dihydro-5-methyl-3(2H)-pyridazinon

Hergestellt analog Beispiel 1 aus 1-[4-(2-Isobutoxy-ethyl)phenoxy]-2,3-epoxypropan und 6-[2-(2-Aminoethyl)benzimidazol-5-yl]-4,5-dihydro-5-methyl-3(2H)-pyridazinon.
Ausbeute: 13 % der Theorie,
amorph

| $C_{29}H_{39}N_5O_4$ (521,7) | | | |
|---|---|---|---|
| Ber.:<br>Gef.: | C 66,77<br>66,59 | H 7,54<br>7,58 | N 13,43<br>13,69 |

$^1$H-NMR-Spektrum ($d_6$-DMSO/$CD_3OD$):
$\delta$ = 0,83 (d,6H); 1,13 (d,3H); 1,78 (m,1H); 2,29 (dd,1H); 2,68-2,80 (m,3H); 3,10-3,20 (m,3H); 3,28 (m,2H); 3,99 (m,2H); 4,20 (m,1H); 6,89 (d,2H); 7,18 (d,2H); 7,45 (d,1H); 7,71 (d,1H); 7,90 (s,1H) ppm.

Beispiel 49

6-[2-[2-[3-(2-Allyl-phenoxy)-2-hydroxypropylamino]ethyl]benzimidazol-5-yl]-4,5-dihydro-5-methyl-3(2H)-pyridazinon

Hergestellt analog Beispiel 1 aus 1-(2-Allyl-phenoxy)-2,3-epoxypropan und 6-[2-(2-Aminoethyl)-benzimidazol-5-yl]-4,5-dihydro-5-methyl-3(2H)-pyridazinon.
Ausbeute: 14 % der Theorie,
amorph

| $C_{26}H_{31}N_5O_3$ (461,6) | | | |
|---|---|---|---|
| Ber.: | C 67,65 | H 6,77 | N 15,18 |
| Gef.: | 67,44 | 6,59 | 15,09 |

$^1$H-NMR-Spektrum ($d_6$-DMSO/$CD_3OD$):
$\delta$ = 1,13 (d,3H); 2,27 (dd,1H); 2,65-2,90 (m,3H); 3,07 (m,4H); 3,34 (dd,2H); 3,48 (m,1H); 3,90-4,05 (m,3H); 4,93-5,11 (m,2H); 5,85-6,07 (m,1H); 6,80-6,97 (m,2H); 7,05-7,20 (m,2H); 7,48 (d,1H); 7,63 (dd,1H); 7,83 (d,1H) ppm.

Beispiel 50

6-[2-[2-[3-(2-Chlor-5-methyl-phenoxy)-2-hydroxypropylamino]ethyl]benzimidazol-5-yl]-4,5-dihydro-5-methyl-3(2H)-pyridazinon

Hergestellt analog Beispiel 1 aus 1-(2-Chlor-5-methyl-phenoxy)-2,3-epoxypropan und 6-[2-(2-Aminoethyl)benzimidazol-5-yl]-4,5-dihydro-5-methyl-3(2H)-pyridazinon.
Ausbeute: 33 % der Theorie,
amorph

| $C_{24}H_{28}ClN_5O_3$ (470,0) | | | | |
|---|---|---|---|---|
| Ber.: | C 61,29 | H 6,00 | N 14,89 | Cl 7,54 |
| Gef.: | 61,00 | 5,87 | 14,55 | 7,55 |

$^1$H-NMR-Spektrum ($d_6$-DMSO/$CD_3OD$):
$\delta$ = 1,13 (d,3H); 2,26 (s,3H); 2,26 (dd,1H); 2,65-2,96 (m,3H); 3,00-3,19 (m,4H); 3,48 (m,1H); 4,00 (m,3H); 6,74 (dd,1H); 6,94 (d,1H); 7,23 (d,1H); 7,49 (d,1H); 7,69 (dd,1H); 7,88 (d,1H) ppm.

Beispiel 51

6-[2-[2-[3-(2-Cyano-phenoxy)-2-hydroxypropylamino]ethyl]benzimidazol-5-yl]-4,5-dihydro-5-methyl-3(2H)-pyridazinon

Hergestellt analog Beispiel 1 aus 1-(2-Cyano-phenoxy)-2,3-epoxypropan und 6-[2-(2-Aminoethyl)-benzimidazol-5-yl]-4,5-dihydro-5-methyl-3(2H)-pyridazinon.
Ausbeute: 29 % der Theorie,
amorph

| $C_{24}H_{26}N_6O_3$ (446,5) | | | |
|---|---|---|---|
| Ber.: | C 64,56 | H 5,87 | N 18,82 |
| Gef.: | 64,43 | 5,81 | 18,59 |

$^1$H-NMR-Spektrum ($d_6$-DMSO/$CD_3OD$):
δ = 1,12 (d,3H); 2,24 (dd,1H); 2,60-2,85 (m,3H); 2,90-3,10 (m,4H); 3,35-3,55 (m,1H); 3,92 (m,1H); 4,02-4,18 (m,2H); 7,08 (t,1H); 7,21 (d,1H); 7,47 (d,1H); 7,55-7,74 (m,3H); 7,83 (d,1H) ppm.

Beispiel 52

6-[2-[3-(3-(Naphthyl-1-oxy)-2-hydroxypropylamino)propyl]benzimidazol-5-yl]-4,5-dihydro-5-methyl-3(2H)-pyridazinon

Hergestellt analog Beispiel 1 aus 1-(Naphthyl-1-oxy)-2,3-epoxypropan und 6-[2-(3-Aminopropyl)-benzimidazol-5-yl]-4,5-dihydro-5-methyl-3(2H)-pyridazinon.
Ausbeute: 32 % der Theorie,
amorph

| $C_{28}H_{31}N_5O_3$ (485,6) | | | |
|---|---|---|---|
| Ber.: | C 69,26 | H 6,44 | N 14,42 |
| Gef.: | 69,10 | 6,21 | 14,07 |

$^1$H-NMR-Spektrum ($d_6$-DMSO/$CD_3OD$):
δ = 1,11 (d,3H); 2,05 (m,2H); 2,22 (dd,1H); 2,70 (dd,1H); 2,80-3,10 (m,6H); 3,35-3,55 (m,1H); 4,05-4,26 (m,3H); 6,95 (dd,1H); 7,35-7,55 (m,5H); 7,65 (dd,1H); 7,85 (m,2H); 8,23 (dd,1H) ppm.

Beispiel 53

6-[2-[2-(3-(2-Cyano-phenoxy)-2-hydroxypropylamino)]ethyl]benzimidazol-5-yl]-3(2H)-pyridazinon

Hergestellt analog Beispiel 1 aus 1-(2-Cyano-phenoxy)-2,3-epoxypropan und 6-[2-(2-Aminoethyl)-benzimidazol-5-yl]-3(2H)-pyridazinon.
Ausbeute: 19 % der Theorie,
Schmelzpunkt: 188-189 °C

| $C_{23}H_{22}N_6O_3$ (430,5) | | | |
|---|---|---|---|
| Ber.: | C 64,17 | H 5,15 | N 19,52 |
| Gef.: | 63,92 | 5,14 | 19,62 |

Beispiel 54

6-[2-[2-(3-(Naphthyl-1-oxy)-2-hydroxypropylamino)]ethyl]benzimidazol-5-yl]-3(2H)-pyridazinon

Hergestellt analog Beispiel 1 aus 1-(Naphthyl-1-oxy)-2,3-epoxypropan und 6-[2-(2-Aminoethyl)-

benzimidazol-5-yl]-3(2H)-pyridazinon.
Ausbeute: 11 % der Theorie,
Schmelzpunkt: 196-197 ° C

| $C_{26}H_{25}N_5O_3$ (455,5) | | | |
|---|---|---|---|
| Ber.: | C 68,56 | H 5,53 | N 15,37 |
| Gef.: | 68,46 | 5,36 | 15,41 |

Beispiel 55

6-[1-[2-[3-(4-(2-Isobutoxy-ethyl)-phenoxy)-2-hydroxypropylamino]ethyl]benzimidazol-5-yl]-4,5-dihydro-5-methyl-3(2H)-pyridazinon

Hergestellt analog Beispiel 1 aus 1-[4-(2-Isobutoxy-ethyl)phenoxy]-2,3-epoxypropan und 6-[1-(2-Aminoethyl)benzimidazol-5-yl]-4,5-dihydro-5-methyl-3(2H)-pyridazinon.
Ausbeute: 18 % der Theorie,
Schmelzpunkt: ab 75 ° C (Sintern)
$R_f$-Wert: 0,63 (Kieselgel, Dichlormethan/Methanol/Ammoniak = 80:20:0,5)

| $C_{29}H_{39}N_5O_4$ (521,6) | | | |
|---|---|---|---|
| Ber.: | C 66,77 | H 7,54 | N 13,43 |
| Gef.: | 66,51 | 7,33 | 13,10 |

Beispiel 56

6-[1-[2-[3-(4-(2-Isobutoxy-ethyl)-phenoxy)-2-hydroxypropylamino-ethyl]benzimidazol-5-yl]-4,5-dihydro-3(2H)-pyridazinon

Hergestellt analog Beispiel 1 aus 1-[4-(2-Isobutoxy-ethyl)phenoxy]-2,3-epoxypropan und 6-[1-(2-Aminoethyl)benzimidazol-5-yl]-4,5-dihydro-3(2H)-pyridazinon.
Ausbeute: 22 % der Theorie,
Schmelzpunkt: 120-122 ° C

| $C_{28}H_{37}N_5O_4$ (507,6) | | | |
|---|---|---|---|
| Ber.: | C 66,25 | H 7,35 | N 13,80 |
| Gef.: | 66,08 | 7,32 | 13,94 |

Beispiel 57

6-[1-[2-[3-(4-(2-Cyclopropylmethoxy-ethyl)phenoxy)-2-hydroxypropylamino]ethyl]benzimidazol-5-yl]-4,5-dihydro-3(2H)-pyridazinon

Hergestellt analog Beispiel 1 aus 1-[4-(2-Cyclopropylmethoxy-ethyl)phenoxy]-2,3-epoxypropan und 6-[1-(2-Aminoethyl)benzimidazol-5-yl]-4,5-dihydro-3(2H)-pyridazinon.
Ausbeute: 28 % der Theorie,
Schmelzpunkt: 150-151 °C

| $C_{28}H_{35}N_5O_4$ (505,6) | | | |
|---|---|---|---|
| Ber.: | C 66,51 | H 6,98 | N 13,85 |
| Gef.: | 66,30 | 7,04 | 13,84 |

Beispiel 58

6-[1-[2-[3-(4-n-Butylsulfonyloxy-phenoxy)-2-hydroxypropylamino]ethyl]benzimidazol-5-yl]-4,5-dihydro-3(2H)-pyridazinon

Hergestellt analog Beispiel 1 aus 1-(4-n-Butylsulfonyloxyphenoxy)-2,3-epoxypropan und 6-[1-(2-Aminoethyl)benzimidazol-5-yl]-4,5-dihydro-3(2H)-pyridazinon.
Ausbeute: 7 % der Theorie,
amorph, $R_f$-Wert: 0,24 (Kieselgel, Dichlormethan/Ethanol = 80:20)

| $C_{26}H_{33}N_5O_6S$ (543,7) | | | | |
|---|---|---|---|---|
| Ber.: | C 57,44 | H 6,12 | N 12,88 | S 5,90 |
| Gef.: | 57,39 | 6,23 | 13,03 | 5,74 |

Beispiel 59

6-[1-[2-[3-(4-Methylsulfonyloxy-phenoxy)-2-hydroxypropylamino]ethyl]benzimidazol-5-yl]-4,5-dihydro-3(2H)-pyridazinon

Hergestellt analog Beispiel 1 aus 1-(4-Methylsulfonyloxyphenoxy)-2,3-epoxypropan und 6-[1-(2-Aminoethyl)benzimidazol-5-yl]-4,5-dihydro-3(2H)-pyridazinon.
Ausbeute: 23 % der Theorie,
amorph

| $C_{23}H_{27}N_5O_6S$ (501,6) | | | | |
|---|---|---|---|---|
| Ber.: | C 55,08 | H 5,43 | N 13,96 | S 6,39 |
| Gef.: | 54,90 | 5,46 | 14,20 | 6,54 |

$^1$H-NMR-Spektrum ($CDCl_3/CD_3OD$):
$\delta$ = 2,58-2,90 (m,4H); 3,03-3,20 (m,3H); 3,15 (s,3H); 3,45-3,65 (m,1H); 3,85-4,10 (m,3H); 4,38 (t,2H); 6,88 (dt,2H); 7,19 (dt,2H); 7,50 (d,1H); 7,85 (dd,1H); 8,04 (d,1H); 8,10 (s,1H) ppm.

Beispiel 60

6-[1-[2-[3-(4-(2-Cyclobutylmethoxy-ethyl)phenoxy)-2-hydroxypropylamino]ethyl]benzimidazol-5-yl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[1-(2-Aminoethyl)benzimidazol-5-yl]-4,5-dihydro-3(2H)-pyridazinon.
Ausbeute: 13 % der Theorie,
Schmelzpunkt: ab 125 °C (Sintern)

| $C_{29}H_{37}N_5O_4$ (519,6) | | | |
|---|---|---|---|
| Ber.: | C 67,03 | H 7,18 | N 13,48 |
| Gef.: | 66,81 | 6,94 | 13,47 |

Beispiel 61

6-[1-[2-[3-(4-(2-Isopropoxyethoxy-methyl)phenoxy)-2-hydroxypropylamino]ethyl]benzimidazol-5-yl]-4,5-dihydro-3(2H)-pyridazinon-dihydrochlorid

Hergestellt analog Beispiel 1 aus 1-[4-(2-Isopropoxyethoxymethyl)phenoxy]-2,3-epoxypropan und 6-[1-(2-Aminoethyl)benzimidazol-5-yl]-4,5-dihydro-3(2H)-pyridazinon. Der erhaltene Rückstand wird in Methanol gelöst, mit etherischer Salzsäure versetzt und das Dihydrochlorid durch Zugabe von Aceton gefällt.
Ausbeute: 13 % der Theorie,
Schmelzpunkt: ab 200 °C (Sintern)

| $C_{28}H_{37}N_5O_5$ x 2 HCl (596,5) | | | | |
|---|---|---|---|---|
| Ber.: | C 56,38 | H 6,59 | N 11,74 | Cl 11,89 |
| Gef.: | 56,19 | 6,56 | 12,00 | 11,72 |

Beispiel 62

6-[1-[3-[3-(4-(2-Isobutoxyethyl)phenoxy)-2-hydroxypropylamino]propyl]benzimidazol-5-yl]-4,5-dihydro-3(2H)-pyridazinon

Hergestellt analog Beispiel 1 aus 1-[4-(2-Isobutoxyethyl)phenoxy]-2,3-epoxypropan und 6-[1-(3-Aminopropyl)benzimidazol-5-yl]-4,5-dihydro-3(2H)-pyridazinon.
Ausbeute: 32 % der Theorie,
Schmelzpunkt: 132-134 °C

| $C_{29}H_{39}N_5O_4$ (521,67) | | | |
|---|---|---|---|
| Ber.: | C 66,77 | H 7,54 | N 13,43 |
| Gef.: | 66,69 | 7,51 | 13,41 |

Beispiel 63

6-[1-[2-[3-(4-(2-Isobutoxyethyl)phenoxy)-2-hydroxypropylamino]-2,2-dimethyl-ethyl]benzimidazol-5-yl]-4,5-dihydro-3(2H)-pyridazinon

Hergestellt analog Beispiel 1 aus 1-[4-(2-Isobutoxyethyl)phenoxy]-2,3-epoxypropan und 6-[1-(2-Amino-2,2-dimethyl-ethyl)benzimidazol-5-yl]-4,5-dihydro-3(2H)-pyridazinon. Ausbeute: 11 % der Theorie, Schmelzpunkt: 130-132 °C

| $C_{30}H_{41}N_5O_4$ (535,7) | | | |
|---|---|---|---|
| Ber.: | C 67,26 | H 7,71 | N 13,07 |
| Gef.: | 66,96 | 7,56 | 13,41 |

Beispiel 64

6-[1-[2-[3-(4-(2-Isopropoxyethoxy-methyl)phenoxy)-2-hydroxypropylamino]-2,2-dimethyl-ethyl]benzimidazol-5-yl]-4,5-dihydro-3(2H)-pyridazinon

Hergestellt analog Beispiel 1 aus 1-[4-(2-Isopropoxyethoxymethyl)phenoxy]-2,3-epoxypropan und 6-[1-(2-Amino-2,2-dimethyl-ethyl)benzimidazol-5-yl]-4,5-dihydro-3(2H)-pyridazinon.
Ausbeute: 9 % der Theorie,
amorph, $R_f$-Wert: 0,55 (Kieselgel, Dichlormethan/Methanol/Ammoniak = 80:20:0,5)

| $C_{30}H_{41}N_5O_5$ (551,7) | | | |
|---|---|---|---|
| Ber.: | C 65,31 | H 7,49 | N 12,70 |
| Gef.: | 65,11 | 7,29 | 12,57 |

Beispiel 65

6-[1-[2-[3-(4-(2-Isobutoxyethyl)phenoxy)-2-hydroxypropylamino]ethyl]-2-methyl-benzimidazol-5-yl]-4,5-dihydro-3(2H)-pyridazinon

Hergestellt analog Beispiel 1 aus 1-[4-(2-Isobutoxyethyl)phenoxy]-2,3-epoxypropan und 6-[1-(2-Aminoethyl)-2-methyl-benzimidazol-5-yl]-4,5-dihydro-3(2H)-pyridazinon.
Ausbeute: 28 % der Theorie,
Schmelzpunkt: 198-200 °C

| $C_{29}H_{39}N_5O_4$ (521,7) | | | |
|---|---|---|---|
| Ber.: | C 66,77 | H 7,54 | N 13,43 |
| Gef.: | 66,56 | 7,53 | 13,30 |

Beispiel 66

6-[1-[2-[3-(4-Isopropylsulfonyloxy-phenoxy)-2-hydroxypropylamino]ethyl]-2-methyl-benzimidazol-5-yl]-4,5-dihydro-3(2H)-pyridazinon

Hergestellt analog Beispiel 1 aus 1-(4-Isopropylsulfonyloxyphenoxy)-2,3-epoxypropan und 6-[1-(2-Aminoethyl)-2-methyl-benzimidazol-5-yl]-4,5-dihydro-3(2H)-pyridazinon.
Ausbeute: 31 % der Theorie,
Schmelzpunkt: 176-178 °C

| $C_{26}H_{33}N_5O_6S$ (543,7) | | | |
|---|---|---|---|
| Ber.:<br>Gef.: | C 57,44<br>57,34 | H 6,12<br>6,07 | N 12,88<br>12,70 |

Beispiel 67

6-[1-[2-[3-(4-(2-Cyclopropylmethoxy-ethyl)phenoxy)-2-hydroxypropylamino]ethyl]-2-methyl-benzimidazol-5-yl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[1-(2-Aminoethyl)-2-methyl-benzimidazol-5-yl]-4,5-dihydro-3(2H)-pyridazinon.
Ausbeute: 21 % der Theorie,
Schmelzpunkt: 188-190 °C

| $C_{29}H_{37}N_5O_4$ (519,7) | | | |
|---|---|---|---|
| Ber.:<br>Gef.: | C 62,68<br>62,59 | H 7,44<br>7,31 | N 12,60<br>12,42 |

Beispiel 68

6-[1-[3-[3-(4-(2-Isobutoxy-ethyl)Phenoxy)-2-hydroxypropylamino]propyl]-2-methyl-benzimidazol-5-yl]-4,5-dihydro-3(2H)-pyridazinon

Hergestellt analog Beispiel 1 aus 1-[4-(2-Isobutoxy-ethyl)phenoxy]-2,3-epoxypropan und 6-[1-(3-Aminopropyl)-2-methyl-benzimidazol-5-yl]-4,5-dihydro-3(2H)-pyridazinon.
Ausbeute: 6 % der Theorie,
amorph, $R_f$-Wert: 0,56 (Kieselgel, Dichlormethan/Methanol/Ammoniak = 80:20:0,5)

| $C_{30}H_{41}N_5O_4$ (535,7) | | | |
|---|---|---|---|
| Ber.:<br>Gef.: | C 67,26<br>67,11 | H 7,72<br>7,53 | N 13,08<br>13,00 |

Beispiel 69

6-[1-[2-[3-(4-(2-Isobutoxy-ethyl)Phenoxy)-2-hydroxypropylamino]ethyl]-2-hydroxy-benzimidazol-5-yl]-4,5-dihydro-3(2H)-pyridazinon

Hergestellt analog Beispiel 1 aus 1-[4-(2-Isobutoxy-ethyl)phenoxy]-2,3-epoxypropan und 6-[1-(2-Aminoethyl)-2-hydroxybenzimidazol-5-yl]-4,5-dihydro-3(2H)-pyridazinon. Ausbeute: 17 % der Theorie, Schmelzpunkt: ab 110° C (Sintern)

| $C_{28}H_{37}N_5O_5$ (523,6) | | | |
|---|---|---|---|
| Ber.: | C 64,22 | H 7,12 | N 13,38 |
| Gef.: | 63,91 | 6,99 | 13,44 |

Beispiel 70

6-[1-[2-[3-(4-(2-Cyclopropylmethoxy-ethyl)phenoxy)-2-hydroxypropylamino]ethyl]-2-hydroxy-benzimidazol-5-yl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[1-(2-Aminoethyl)-2-hydroxy-benzimidazol-5-yl]-4,5-dihydro-3(2H)-pyridazinon.
Ausbeute: 13 % der Theorie,
amorph, $R_f$-Wert: 0,56 (Kieselgel, Dichlormethan/Methanol/Ammoniak = 80:20:0,5)

| $C_{28}H_{35}N_5O_5$ (521,6) | | | |
|---|---|---|---|
| Ber.: | C 64,47 | H 6,76 | N 13,43 |
| Gef.: | 64,31 | 6,39 | 13,55 |

Beispiel 71

6-[1-[2-[3-(4-Isopropylsulfonyloxy-phenoxy)-2-hydroxypropylamino]ethyl]-2-hydroxy-benzimidazol-5-yl]-4,5-dihydro-3(2H)-pyridazinon

Hergestellt analog Beispiel 1 aus 1-(4-Isopropylsulfonyloxyphenoxy)-2,3-epoxypropan und 6-[1-(2-Aminoethyl)-2-hydroxybenzimidazol-5-yl]-4,5-dihydro-3(2H)-pyridazinon.
Ausbeute: 18 % der Theorie,
amorph

| $C_{25}H_{31}N_5O_7S$ (545,6) | | | | |
|---|---|---|---|---|
| Ber.: | C 55,03 | H 5,73 | N 12,84 | S 5,88 |
| Gef.: | 54,83 | 5,44 | 12,29 | 5,89 |

$^1$H-NMR-Spektrum ($d_6$-DMSO/$CD_3OD$):
$\delta$ = 1,42 (d,6H); 2,42 (t,2H); 2,55-2,97 (m,6H); 3,66 (m,1H); 3,74-3,96 (m,5H); 6,93 (d,2H); 7,13-7,25 (m,3H); 7,27-7,43 (m,2H) ppm.

Beispiel 72

6-[1-[2-[3-(4-(2-Isobutoxyethyl)phenoxy)-2-hydroxypropylamino]ethyl]benztriazol-5-yl]-4,5-dihydro-3(2H)-pyridazinon

Hergestellt analog Beispiel 1 aus 1-[4-(2-Isobutoxyethyl)phenoxy]-2,3-epoxypropan und 6-[1-(2-Aminoethyl)benztriazol-5-yl]-4,5-dihydro-3(2H)-pyridazinon.
Ausbeute: 19 % der Theorie,
amorph

| $C_{27}H_{36}N_6O_4$ (508,6) | | | |
|---|---|---|---|
| Ber.: | C 63,76 | H 7,13 | N 16,53 |
| Gef.: | 63,59 | 7,13 | 16,44 |

$^1$H-NMR-Spektrum ($d_6$-DMSO/$CD_3OD$):
$\delta$ = 0,83 (d,6H); 1,77 (m,1H); 2,55-2,80 (m,4H); 3,03-3,18 (m,7H); 3,50 (t,2H); 3,75 (m,4H); 4,78 (m,2H); 6,73 (d,2H); 7,10 (d,2H); 7,90 (d,1H); 8,06 (dd,1H); 8,31 (d,1H) ppm.

Beispiel 73

6-[1-[2-[3-(4-(2-Cyclobutylmethoxy-ethyl)phenoxy)-2-hydroxypropylamino]ethyl]benztriazol-5-yl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[1-(2-Aminoethyl)benz triazol-5-yl]-4,5-dihydro-3(2H)-pyridazinon. Ausbeute: 17 % der Theorie,
Schmelzpunkt: 157-158 °C

| $C_{28}H_{36}N_6O_4$ (520,6) | | | |
|---|---|---|---|
| Ber.: | C 64,60 | H 6,97 | N 16,14 |
| Gef.: | 64,50 | 7,04 | 16,22 |

Beispiel

6-[1-[2-[3-(4-Methylsulfonyloxy-phenoxy)-2-hydroxypropylamino]ethyl]benztriazol-5-yl]-4,5-dihydro-3(2H)-pyridazinon

Hergestellt analog Beispiel 1 aus 1-(4-Methylsulfonyloxyphenoxy)-2,3-epoxypropan und 6-[1-(2-Aminoethyl)benztriazol-5-yl]-4,5-dihydro-3(2H)-pyridazinon.
Ausbeute: 23 % der Theorie,
Schmelzpunkt: 156-158 °C

| $C_{22}H_{26}N_6O_6S$ (502,6) | | | | |
|---|---|---|---|---|
| Ber.: | C 52,58 | H 5,21 | N 16,72 | S 6,38 |
| Gef.: | 52,30 | 5,22 | 16,85 | 6,45 |

Beispiel

6-[1-[2-[3-(4-n-Butylsulfonyloxy-phenoxy)-2-hydroxypropylamino]ethyl]benztriazol-5-yl]-4,5-dihydro-3(2H)-pyridazinon

Hergestellt analog Beispiel 1 aus 1-(4-n-Butylsulfonyloxyphenoxy)-2,3-epoxypropan und 6-[1-(2-Aminoethyl)benztriazol-5-yl]-4,5-dihydro-3(2H)-pyridazinon.
Ausbeute: 8 % der Theorie,
amorph, $R_f$-Wert: 0,49 (Kieselgel, Dichlormethan/Ethanol = 9:1)

| $C_{25}H_{32}N_6O_6S$ (544,6) | | | | |
|---|---|---|---|---|
| Ber.: | C 55,13 | H 5,92 | N 15,43 | S 5,89 |
| Gef.: | 54,99 | 5,51 | 15,30 | 6,02 |

Beispiel 76

6-[1-[2-[3-(4-(2-Isobutoxy-ethyl)phenoxy)-2-hydroxypropylamino]ethyl]benzimidazol-5-yl]-3(2H)-pyridazinon

Hergestellt analog Beispiel 1 aus 1-[4-(2-Isobutoxy-ethyl)phenoxy]-2,3-epoxypropan und 6-[1-(2-Aminoethyl)benzimidazol-5-yl]-3(2H)-pyridazinon.
Ausbeute: 8 % der Theorie,
amorph

| $C_{28}H_{35}N_5O_4$ (505,6) | | | |
|---|---|---|---|
| Ber.: | C 64,23 | H 7,12 | N 13,37 |
| Gef.: | 64,07 | 7,29 | 13,14 |

$^1$H-NMR-Spektrum ($d_6$-DMSO/$CD_3OD$):
$\delta$ = 0,80 (d,6H); 1,76 (m,1H); 2,53-2,70 (m,4H); 2,92-3,18 (m,4H); 3,39-3,55 (t,2H); 3,66-3,87 (m,3H); 4,25-4,43 (m,2H); 6,63-7,18 (m,5H); 7,67-7,83 (m,2H); 8,04-8,19 (m,2H); 8,28 (s,1H) ppm.

Beispiel 77

6-[1-[2-[3-(4-(2-Cyclopropylmethoxy-ethyl)phenoxy)-2-hydroxypropylamino]ethyl]benzimidazol-5-yl]-3(2H)-pyridazinon

Hergestellt analog Beispiel 1 aus 1-[4-(2-Cyclopropylmethoxyethyl)phenoxy]-2,3-epoxypropan und 6-[1-(2-Aminoethyl)benzimidazol-5-yl]-3(2H)-pyridazinon.
Ausbeute: 18 % der Theorie,
Schmelzpunkt: 115-117 °C

| $C_{28}H_{33}N_5O_4$ (503,6) | | | |
|---|---|---|---|
| Ber.: | C 66,78 | H 6,61 | N 13,91 |
| Gef.: | 66,77 | 6,41 | 13,74 |

Beispiel 78

6-[1-[2-[3-(4-(2-Cyclobutylmethoxy-ethyl)phenoxy)-2-hydroxypropylamino]ethyl]benzimidazol-5-yl]-3(2H)-pyridazinon

Hergestellt analog Beispiel 1 aus 1-[4-(2-Cyclobutylmethoxyethyl)phenoxy]-2,3-epoxypropan und 6-[1-(2-Aminoethyl)benzimidazol-5-yl]-3(2H)-pyridazinon.
Ausbeute: 13 % der Theorie,
amorph

| $C_{29}H_{35}N_5O_4$ (517,6) | | | |
|---|---|---|---|
| Ber.: | C 67,29 | H 6,82 | N 13,53 |
| Gef.: | 67,00 | 6,71 | 13,52 |

$^1$H-NMR-Spektrum ($d_6$-DMSO/$CD_3OD$):
$\delta$ = 1,60-2,04 (m,7H); 2,38-2,55 (m,2H); 2,71 (t,2H); 3,32 (t,2H); 3,51 (t,2H); 3,93-4,05 (m,2H); 4,27-4,46 (m,3H); 4,54 (t,2H); 6,82 (d,2H); 7,00-7,18 (m,3H); 7,73 (d,1H); 7,86 (dd,1H); 8,11 (d,1H); 8,20 (d,1H); 8,30 (s,1H) ppm.

Beispiel 79

6-[4-[3-[3-(4-(2-Isobutoxyethyl)phenoxy)-2-hydroxypropylamino]propylsulfonyl]phenyl]-4,5-dihydro-3(2H)-pyridazinon

Hergestellt analog Beispiel 1 aus 1-[4-(2-Isobutoxyethyl) phenoxy]-2,3-epoxypropan und 6-[4-(3-Aminopropylsulfonyl]phenyl]-4,5-dihydro-3(2H)-pyridazinon.
Ausbeute: 24 % der Theorie,
Schmelzpunkt: 129-131 °C

| $C_{28}H_{39}N_3O_6S$ (545,7) | | | | |
|---|---|---|---|---|
| Ber.: | C 61,63 | H 7,20 | N 7,70 | S 5,88 |
| Gef.: | 61,43 | 6,95 | 7,53 | 6,07 |

Beispiel 80

6-[4-[3-[3-(4-(2-Cyclopropylmethoxyethyl)phenoxy)-2-hydroxypropylamino]propylsulfonyl]phenyl]-4,5-dihydro-3(2H)-pyridazinon

Hergestellt analog Beispiel 1 aus 1-[4-(2-Cyclopropylmethoxyethyl)phenoxy]-2,3-epoxypropan und 6-[4-(3-Aminopropylsulfonyl)phenyl]-4,5-dihydro-3(2H)-pyridazinon.
Ausbeute: 24 % der Theorie,
Schmelzpunkt: 128-130 °C

| $C_{28}H_{37}N_3O_6S$ (543,69) | | | | |
|---|---|---|---|---|
| Ber.: | C 61,86 | H 6,86 | N 7,73 | S 5,90 |
| Gef.: | 61,75 | 7,09 | 7,60 | 6,06 |

Beispiel 81

6-[4-[3-[3-(4-(2-Isopropoxyethoxymethyl)phenoxy)-2-hydroxypropylamino]propylsulfonyl]phenyl]-4,5-dihydro-3(2H)-pyridazinon

Hergestellt analog Beispiel 1 aus 1-[4-(2-Isopropoxyethoxymethyl)phenoxy]-2,3-epoxypropan und 6-[4-(3-Aminopropylsulfonyl)phenyl]-4,5-dihydro-3(2H)-pyridazinon.
Ausbeute: 23 % der Theorie,
Schmelzpunkt: 108-110 ° C

| $C_{28}H_{39}N_3O_7S$ (561,7) | | | | |
|---|---|---|---|---|
| Ber.: | C 59,87 | H 7,00 | N 7,48 | S 5,71 |
| Gef.: | 59,76 | 6,81 | 7,56 | 5,99 |

Beispiel 82

6-[4-[2-[3-(Naphthyl-1-oxy)-2-hydroxypropylamino]ethyl]phenyl]-4,5-dihydro-3(2H)-pyridazinon-hydrochlorid

Hergestellt analog Beispiel 61 aus 1-(Naphthyl-1-oxy)-2,3-epoxypropan und 6-[4-(2-Aminoethyl)phenyl]-4,5-dihydro-3(2H)-pyridazinon.
Ausbeute: 33 % der Theorie,
Schmelzpunkt: 177-180 ° C

| $C_{25}H_{27}N_3O_3$ x HCl (454,0) | | | | |
|---|---|---|---|---|
| Ber.: | C 66,14 | H 6,22 | N 9,26 | S 7,81 |
| Gef.: | 65,99 | 6,21 | 9,35 | 7,76 |

Beispiel 83

6-[4-[2-[3-(4-(2-Cyclobutylmethoxyethyl)phenoxy)-2-hydroxypropylamino]ethyl]phenyl]-4,5-dihydro-3(2H)-pyridazinon

Hergestellt analog Beispiel 1 aus 1-[4-(2-Cyclobutylmethoxyethyl)phenoxy]-2,3-epoxypropan und 6-[4-(2-Aminoethyl)phenyl]-4,5-dihydro-3(2H)-pyridazinon.
Ausbeute: 24 % der Theorie,
Schmelzpunkt: ab 119 ° C (Sintern)

| $C_{28}H_{37}N_3O_4$ (479,6) | | | |
|---|---|---|---|
| Ber.: | C 70,12 | H 7,78 | N 8,76 |
| Gef.: | 69,94 | 7,79 | 8,67 |

Beispiel 84

6-[4-[2-[3-(Naphthyl-1-oxy)-2-hydroxypropylamino]ethyl]phenyl]-3(2H)-pyridazinon-hydrochlorid

Hergestellt analog Beispiel 61 aus 1-(Naphthyl-1-oxy)-2,3-epoxypropan und 6-[4-(2-Aminoethyl)Phenyl]-3(2H)-pyridazinon.
Ausbeute: 16 % der Theorie,
Schmelzpunkt: 219-222 °C

| $C_{25}H_{25}N_3O_3$ x HCl (452,0) | | | | |
|---|---|---|---|---|
| Ber.: | C 66,43 | H 5,80 | N 9,30 | Cl 7,84 |
| Gef.: | 66,42 | 5,97 | 9,30 | 8,01 |

Beispiel 85

6-[4-[2-[3-(4-(2-Cyclobutylmethoxyethyl)phenoxy)-2-hydroxypropylamino]ethyl]phenyl]-3(2H)-pyridazinon

Hergestellt analog Beispiel 1 aus 1-[4-(2-Cyclobutylmethoxyethyl)phenoxy]-2,3-epoxypropan und 6-[4-(2-Aminoethyl)phenyl]-3(2H)-pyridazinon.
Ausbeute: 10 % der Theorie,
Schmelzpunkt: 119-121 °C

| $C_{28}H_{35}N_3O_4$ (477,6) | | | |
|---|---|---|---|
| Ber.: | C 70,42 | H 7,39 | N 8,80 |
| Gef.: | 70,31 | 7,39 | 8,76 |

Beispiel 86

6-[4-[2-[3-(Naphthyl-1-oxy)-2-hydroxypropylamino]ethylaminocarbonylmethyl]phenyl]-4,5-dihydro-3(2H)-pyridazinon-hydrochlorid

Hergestellt analog Beispiel 61 aus 1-(Naphthyl-1-oxy)-2,3-epoxypropan und 6-[4-((2-Aminoethyl)-aminocarbonylmethyl)phenyl]-3(2H)-pyridazinon.
Ausbeute: 12 % der Theorie,
amorph

| $C_{27}H_{30}N_4O_4$ x HCl (511,1) | | | | |
|---|---|---|---|---|
| Ber.: | C 63,45 | H 6,11 | N 10,96 | Cl 6,94 |
| Gef.: | 63,60 | 6,25 | 10,75 | 7,03 |

$^1$H-NMR-Spektrum ($d_6$-DMSO/$CD_3OD$):
$\delta$ = 2,40-2,55 (m,2H); 2,88-3,57 (m,9H); 4,08-4,22 (m,3H); 4,32 (m,1H); 6,98 (dd,1H); 7,29-7,73 (m,8H); 7,83-7,91 (m,1H); 8,22-8,35 (m,1H) ppm.

Beispiel 87

6-[4-[2-[3-(4-(2-Cyclobutylmethoxyethyl)phenoxy-2-hydroxypropylamino]ethylaminocarbonylmethyl]phenyl]-4,5-dihydro-3(2H)-pyridazinon-hydrochlorid

Hergestellt analog Beispiel 61 aus 1-[4-(2-Cyclobutylmethoxyethyl)phenoxy]-2,3-epoxypropan und 6-[4-((2-Aminoethyl)aminocarbonylmethyl)phenyl]-4,5-dihydro-3(2H)-pyridazinon.
Ausbeute: 12 % der Theorie,
Schmelzpunkt: 214-216 °C

| $C_{30}H_{40}N_4O_5$ x HCl (573,1) | | | |
|---|---|---|---|
| Ber.: | C 62,87 | H 7,21 | N 9,78 |
| Gef.: | 62,75 | 7,03 | 9,77 |

Beispiel 88

6-[3-[2-[3-(4-(2-Cyclobutylmethoxyethyl)phenoxy)-2-hydroxypropylamino]ethoxyphenyl]-3(2H)-pyridazinon

Hergestellt analog Beispiel 1 aus 1-[4-(2-Cyclobutylmethyloxyethyl)phenoxy]-2,3-epoxypropan und 6-[3-(2-Aminoethoxy)phenyl]-3(2H)-pyridazinon.
Ausbeute: 11 % der Theorie,
Schmelzpunkt: 111-112 °C

| $C_{28}H_{35}N_3O_5$ (493,6) | | | |
|---|---|---|---|
| Ber.: | C 68,13 | H 7,15 | N 8,51 |
| Gef.: | 67,95 | 7,10 | 8,50 |

Beispiel 89

6-[4-[2-[3-(4-(2-Isopropoxyethoxy-methyl)phenoxy)-2-hydroxypropylamino]ethoxy]-3-methyl-phenyl]-3(2H)-pyridazinon

Hergestellt analog Beispiel 1 aus 1-[4-(2-Isopropoxyethoxymethyl)phenoxy]-2,3-epoxypropan und 6-[4-(2-Aminoethoxy)-3-methyl-phenyl]-3(2H)-pyridazinon.
Ausbeute: 35 % der Theorie,
Schmelzpunkt: 121-122 °C

| $C_{28}H_{37}N_3O_6$ (511,6) | | | |
|---|---|---|---|
| Ber.: | C 65,73 | H 7,29 | N 8,21 |
| Gef.: | 65,51 | 7,14 | 8,10 |

Beispiel 90

6-[4-[2-[3-(4-(2-phenoxyethoxy-methyl)phenoxy)-2-hydroxypropylamino]ethoxy]-3-chlor-phenyl]-4,5-dihydro-3(2H)-pyridazinon

Hergestellt analog Beispiel 1 aus 1-[4-(2-Phenoxyethoxymethyl)phenoxy]-2,3-epoxypropan und 6-[4-(2-Aminoethoxy-3-chlor-phenyl]-4,5-dihydro-3(2H)-pyridazinon.
Ausbeute: 28 % der Theorie,
Schmelzpunkt: 95-97 °C

| $C_{30}H_{34}ClN_3O_6$ (568,10) | | | | |
|---|---|---|---|---|
| Ber.: | C 63,43 | H 6,03 | N 7,40 | Cl 6,24 |
| Gef.: | 63,35 | 5,97 | 7,44 | 6,44 |

Beispiel 91

6-[4-[2-[3-(4-(2-Phenoxyethoxy-methyl)phenoxy)-2-hydroxypropylamino]ethoxy]-3-methyl-phenyl]-4,5-dihydro-3(2H)-pyridazinon

Hergestellt analog Beispiel 1 aus 1-[4-(2-Phenoxyethoxymethyl)phenoxy]-2,3-epoxypropan und 6-[4-(2-Aminoethoxy3-methyl-phenyl]-4,5-dihydro-3(2H)-pyridazinon.
Ausbeute: 26 % der Theorie,
Schmelzpunkt: 85-87 °C

| $C_{31}H_{37}N_3O_6$ (547,70) | | | |
|---|---|---|---|
| Ber.: | C 67,98 | H 6,81 | N 7,67 |
| Gef.: | 67,89 | 7,01 | 7,81 |

Beispiel 92

6-[4-[2-[3-(4-(2-n-Butyloxyethoxy-methyl)phenoxy)-2-hydroxypropylamino]ethoxy]-3-methyl-phenyl]-4,5-dihydro-3(2H)-pyridazinon

Hergestellt analog Beispiel 1 aus 1-[4-(2-n-Butyloxyethoxy-methyl)phenoxy]-2,3-epoxypropan und 6-[4-(2-Aminoethoxy-3-methyl-phenyl]-4,5-dihydro-3(2H)-pyridazinon.
Ausbeute: 28 % der Theorie,
Schmelzpunkt: 96-98 °C

| $C_{29}H_{41}N_3O_6$ (527,70) | | | |
|---|---|---|---|
| Ber.: | C 66,00 | H 7,83 | N 7,96 |
| Gef.: | 65,88 | 7,95 | 7,80 |

Beispiel 93

6-[4-[2-[3-(4-(2-n-Butyloxyethoxy-methyl)phenoxy)-2-hydroxypropylamino]ethoxy]-3-chlor-phenyl]-4,5-dihydro-3(2H)-pyridazinon

Hergestellt analog Beispiel 1 aus 1-[4-(2-n-Butyloxyethoxy-methyl)phenoxy]-2,3-epoxypropan und 6-[4-(2-Aminoethoxy-3-chlor-phenyl]-4,5-dihydro-3(2H)-pyridazinon.
Ausbeute: 27 % der Theorie,
Schmelzpunkt: 108-110 °C

| $C_{28}H_{38}ClN_3O_6$ (548,10) | | | | |
|---|---|---|---|---|
| Ber.: | C 61,36 | H 6,99 | N 7,67 | Cl 6,47 |
| Gef.: | 61,51 | 6,85 | 7,61 | 6,54 |

Beispiel 94

6-[4-[2-[3-(4-(2-n-Butyloxyethoxy-methyl)phenoxy)-2-hydroxypropylamino]ethoxy]phenyl]-4.5-dihydro-3(2H)-pyridazinon

Hergestellt analog Beispiel 1 aus 1-[4-(2-n-Butyloxyethoxy-methyl)phenoxy]-2,3-epoxypropan und 6-[4-(2-Aminoethoxy)phenyl]-4,5-dihydro-3(2H)-pyridazinon.
Ausbeute: 30 % der Theorie,
Schmelzpunkt: 82-84 °C

| $C_{28}H_{39}N_3O_6$ (513,60) | | | |
|---|---|---|---|
| Ber.: | C 65,48 | H 7,65 | N 8,18 |
| Gef.: | 65,31 | 7,74 | 8,16 |

Beispiel 95

6-[4-[2-[3-(4-(2-Methoxyethoxy-methyl)phenoxy)-2-hydroxypropylamino]ethoxy]-3-methyl-phenyl]-3(2H)-pyridazinon

Hergestellt analog Beispiel 1 aus 1-[4-(2-Methoxyethoxy-methyl)phenoxy]-2,3-epoxypropan und 6-[4-(2-Aminoethoxy)-3-methyl-phenyl]-3(2H)-pyridazinon.
Ausbeute: 32 % der Theorie,
Schmelzpunkt: 127-128 °C

| $C_{26}H_{33}N_3O_6$ (483,55) | | | |
|---|---|---|---|
| Ber.: | C 64,58 | H 6,88 | N 8,69 |
| Gef.: | 64,42 | 6,71 | 8,44 |

In den nachfolgenden pharmazeutischen Anwendungsbeispielen kann als Wirksubstanz jede geeignete Verbindung der Formel I eingesetzt werden, z.B. die Verbindungen der vorstehenden Beispiele:

Beispiel 96

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel 97

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel 98

| Dragées, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel 99

| Dragées, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45° C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel 100

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel 101

| Orale Suspension, enthaltend 50 mg Wirkstoff pro 5 ml | | |
|---|---|---|
| Wirkstoff | | 50,0 mg |
| Hydroxyethylcellulose | | 50,0 mg |
| Sorbinsäure | | 5,0 mg |
| Sorbit 70%ig | | 600,0 mg |
| Glycerin | | 200,0 mg |
| Aroma | | 15,0 mg |
| Wasser | ad | 5,0 ml |

Herstellung:

Destilliertes Wasser wird auf 70° C erhitzt. Hierin wird unter Rühren Hydroxyethylcellulose gelöst. Durch Zugabe von Sorbitlösung und Glycerin wird auf Raumtemperatur abgekühlt.

Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel 102

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40° C wird die gemahlene Wirksubstanz in der Schmelze homogen dispergiert. Es wird auf 38° C abgekühlt und in schwach vorgekühlte Suppositorienformen ausgegossen.

**Ansprüche**

1. 2-Hydroxy-n-propylamine der Formel

$R_1$-O-$CH_2$-CHOH-$CH_2$-NH-A-$R_2$ ,(I)

in der

$R_1$ eine Naphthylgruppe, eine über den Phenylring gebundene 3,4-Dihydro-carbostyrilgruppe, eine durch eine Alkylsulfonyloxygruppe mit 1 bis 5 Kohlenstoffatomen, durch eine Allyl-, Allyloxy-, Cyano- oder Aminocarbonylmethylgruppe substituierte Phenylgruppe, eine durch eine Alkylgruppe mit 1 bis 3 Kohlenstoffatomen substituierte Phenylgruppe, in welcher der Alkylteil in 1-, 2- oder 3-Stellung durch eine Alkoxy-, Cycloalkoxy-, Cycloalkylmethoxy-, 2-Alkoxyethoxy-, 2-Cycloalkoxyethoxy-, 2-Cycloalkylmethoxyethoxy- oder 2-Phenoxyethoxygruppe substituiert ist, wobei der Alkoxyteil jeweils 1 bis 6 Kohlenstoffatome und der Cycloalkyl- oder Cycloalkoxyteil jeweils 3 bis 6 Kohlenstoffatome enthalten kann, oder eine durch eine Alkylgruppe mit 1 bis 3 Kohlenstoffatomen und durch ein Halogenatom disubstituierte Phenylgruppe,

A eine gegebenenfalls durch eine oder zwei Alkylgruppen mit jeweils 1 bis 3 Kohlenstoffatomen substituierte geradkettige Alkylengruppe mit 2 bis 4 Kohlenstoffatomen und

$R_2$ eine Gruppe der Formel

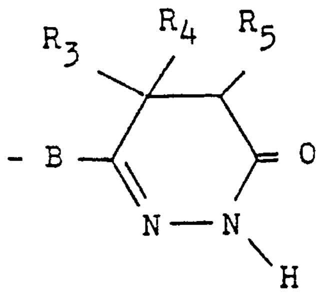

bedeuten, in welcher
$R^3$ ein Wasserstoffatom oder eine. Alkylgruppe mit 1 bis 3 Kohlenstoffatomen,
$R_4$ und $R_5$ jeweils ein Wasserstoffatom oder zusammen eine weitere Kohlenstoff-Kohlenstoffbindung und
B eine Gruppe der Formeln

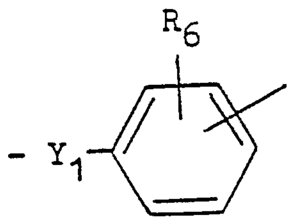

,

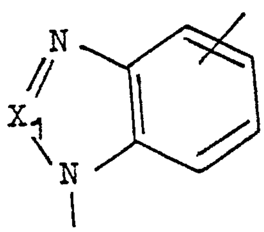

oder

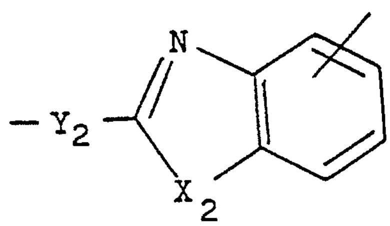

darstellen, in welchen
$R_6$ eine Alkylgruppe mit 1 bis 3 Kohlenstoffatomen, ein Wasserstoff- oder Halogenatom,
$Y_1$ eine Bindung, ein Sauerstoffatom, eine Sulfonylgruppe oder eine $-NH-CO-CH_2$-Gruppe, wobei die $-CH_2$-Gruppe mit dem Phenylkern verbunden ist,
$X_1$ ein Stickstoffatom oder eine gegebenenfalls durch eine Hydroxygruppe oder eine Alkylgruppe mit 1 bis 3 Kohlenstoffatomen substituierte Methingruppe,
$X_2$ ein Sauerstoffatom oder eine Iminogruppe und
$Y_2$ eine Bindung oder eine Iminogruppe bedeuten,
deren Tautomere, deren Enantiomere und deren Säureadditionssalze.

2. 2-Hydroxy-n-propylamine der Formel I gemäß Anspruch 1, in der
$R_1$ eine Naphthyl-, 3,4-Dihydro-carbostyril-(5)-, Allylphenyl-, Allyloxyphenyl-, Cyanophenyl-, Aminocarbonylmethylphenyl- oder Chlor-methylphenylgruppe, eine Alkylsulfonyloxyphenylgruppe mit 1 bis 4 Kohlenstoffatomen im Alkylteil oder eine in 4-Stellung durch einen 2-Alkoxyethyl-, 2-Cycloalkylmethoxyethyl-, 2-Alkoxyethoxymethyl-, 2-Cycloalkoxyethoxymethyl-, 2-Cycloalkylmethoxyethoxymethyl- oder 2-Phenoxyethoxymethylrest substituierte Phenylgruppe, in welcher der Alkoxyteil jeweils 1 bis 4 Kohlenstoffatome und der Cycloalkyloder Cycloalkoxyteil jeweils 3 oder 4 Kohlenstoffatome enthalten kann,
A eine gegebenenfalls in α-Stellung zu dem benachbarten Stickstoffatom durch eine Methylgruppe mono- oder disubstituierte n-Alkylengruppe mit 2 bis 4 Kohlenstoffatomen und $R_2$ eine Gruppe der Formel

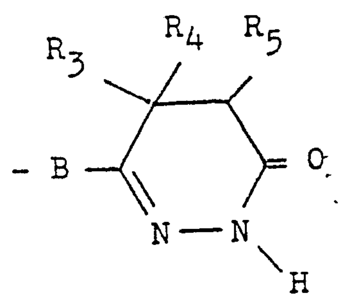

bedeuten, in welcher
$R_3$ ein Wasserstoffatom oder eine Methylgruppe,
$R_4$ und $R_5$ jeweils ein Wasserstoffatom oder zusammen eine weitere. Kohlenstoff-Kohlenstoffbindung und
B eine Gruppe der Formeln

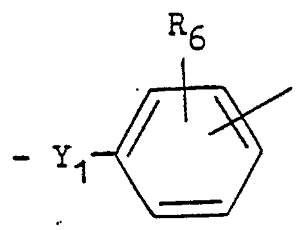

,

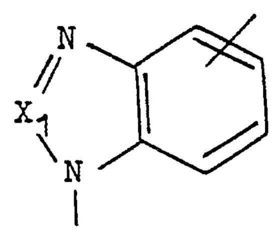

oder

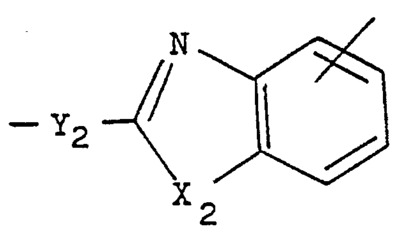

darstellen, in welchen
$R_6$ ein Wasserstoff-, Fluor-, Chlor- oder Bromatom oder eine Methylgruppe,
$Y_1$ eine Bindung, ein Sauerstoffatom oder eine -NH-CO-$CH_2$-Gruppe, wobei die -$CH_2$-Gruppe mit dem Phenylkern verbunden ist,
$X_1$ ein Stickstoffatom, eine gegebenenfalls durch eine Hydroxy- oder Methylgruppe substituierte Methingruppe,
$X_2$ ein Sauerstoffatom oder eine Iminogruppe und

$Y_2$ eine Bindung oder eine Iminogruppe bedeuten, deren Tautomere, deren Enantiomere und deren Säureadditionssalze.

3. 2-Hydroxy-n-propylamine der Formel I gemäß Anspruch 1, in der

$R_1$ eine Naphthyl-, 3,4-Dihydro-carbostyril-(5)-, Allylphenyl-, Allyloxyphenyl- oder Chlor-methylphenylgruppe oder eine in 4-Stellung durch einen 2-Alkoxyethyl-, 2-Cycloalkylmethoxyethyl-, 2-Alkoxyethoxymethyl-, 2-Cycloalkoxyethoxymethyl-, 2-Cycloalkylmethoxyethoxymethyl- oder 2-Phenoxyethoxymethylrest substituierte Phenylgruppe, in welcher der Alkoxyteil jeweils 1 bis 3 Kohlenstoffatome und der Cycloalkyl- oder Cycloalkoxyteil jeweils 3 oder 4 Kohlenstoffatome enthalten kann,

A eine gegebenenfalls in $\alpha$-Stellung zu dem benachbarten Stickstoffatom durch Methylgruppen disubstituierte n-Alkylengruppe mit 2 bis 4 Kohlenstoffatomen und

$R_2$ eine Gruppe der Formel

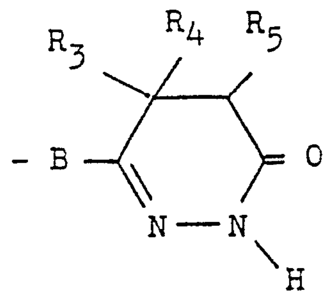

bedeuten, in welcher

$R_3$ ein Wasserstoffatom oder eine Methylgruppe,

$R_4$ und $R_5$ jeweils ein Wasserstoffatom oder zusammen eine weitere Kohlenstoff-Kohlenstoffbindung und

B eine Gruppe der Formeln

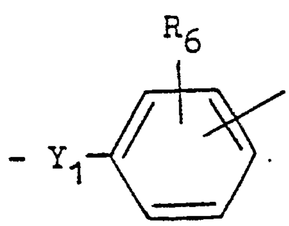

,

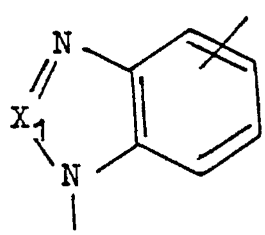

oder

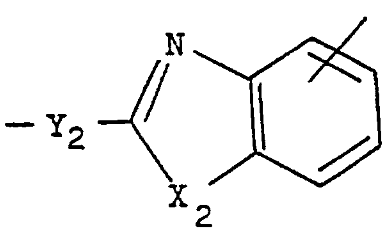

darstellen, in welchen
$R_6$ eine Methylgruppe, ein Wasserstoff- oder Chloratom,
$Y_1$ ein Sauerstoffatom,
$X_1$ eine Methingruppe,
$X_2$ ein Sauerstoffatom oder eine Iminogruppe und
$Y_2$ eine Bindung oder eine Iminogruppe bedeuten,
deren Tautomere, deren Enantiomere und deren Säureadditionssalze.

4. 2-Hydroxy-n-propylamine der Formel I gemäß Anspruch 1:
6-[1-[2-[3-(4-(2-Cyclobutylmethoxy-ethyl)phenoxy)-2-hydroxypropylamino]ethyl]benzimidazol-5-yl]-4,5-dihydro-3(2H)-pyridazinon,
6-[1-[2-[3-(4-(2-Cyclopropylmethoxy-ethyl)phenoxy)-2-hydroxypropylamino]ethyl]benzimidazol-5-yl]-4,5-dihydro-3(2H)-pyridazinon,
6-[4-[2-[3-(4-(2-Cyclopropylmethoxy-ethyl)phenoxy)-2-hydroxypropylamino]ethoxy]phenyl]-4,5-dihydro-3-(2H)-pyridazinon,
6-[4-[2-[3-(4-(2-Cyclopropylmethoxyethyl)phenoxy)-2-hydroxypropylamino]ethoxy]phenyl]-3(2H)-pyridazinon,
6-[4-[2-[3-(4-(2-Cyclobutylmethoxy-ethyl)phenoxy)-2-hydroxypropylamino]ethoxy]phenyl]-4,5-dihydro-3(2H)-pyridazinon,
6-[4-[2-[3-(4-((2-Isopropoxyethoxy)methyl)phenoxy)-2-hydroxypropyl-amino]ethoxy]phenyl]-4,5-dihydro-3-(2H)-pyridazinon,
6-[4-[2-[3-(4-(2-Isopropoxyethoxymethyl)phenoxy)-2-hydroxypropylamino]ethoxy]phenyl]-3(2H)-pyridazinon,
6-[1-[3-[3-(4-(2-Isobutoxyethyl)phenoxy)-2-hydroxypropylamino]propyl]benzimidazol-5-yl]-4,5-dihydro-3(2H)-pyridazinon,
6-[1-[2-[3-(4-(2-Isobutoxyethyl)phenoxy)-2-hydroxypropylamino]-2,2-dimethyl-ethyl]benzimidazol-5-yl]-4,5-dihydro-3(2H)-pyridazinon,
6-[4-[3-[3-(4-(2-Isopropoxyethoxy-methyl)phenoxy)-2-hydroxypropylamino]propoxy]-3-chlor-phenyl]-4,5-dihydro-3(2H)-pyridazinon,
6-[4-[2-[3-(4-(2-Isopropoxyethoxy-methyl)phenoxy)-2-hydroxypropylamino]ethoxy]-3-chlor-phenyl]-4,5-dihydro-3(2H)-pyridazinon,
6-[4-[2-[3-(4-(2-Cyclopropylmethoxy-ethyl)phenoxy)-2-hydroxypropylamino]ethoxy]-3-methyl-phenyl]-4,5-dihydro-3(2H)-pyridazinon,
6-[4-[2-[3-(4-(2-Isopropoxyethoxy-methyl)phenoxy)-2-hydroxypropylamino]ethoxy]-3-methyl-phenyl]-4,5-dihydro-3(2H)-pyridazinon,
6-[4-[3-[3-(4-(2-Cyclopropylmethoxy-ethyl)phenoxy)-2-hydroxypropylamino]propoxy]-3-methyl-phenyl]-4,5-dihydro-3(2H)-pyridazinon,
6-[4-[3-[3-(4-(2-Cyclobutylmethoxy-ethyl)phenoxy)-2-hydroxypropylamino]propoxy]-3-methyl-phenyl]-4,5-dihydro-3(2H)-pyridazinon,
6-[4-[2-[3-(4-(2-Isopropoxyethoxy-methyl)phenoxy)-2-hydroxypropylamino]ethoxy]-3-methyl-phenyl]-3(2H)-pyridazinon,
6-[4-[2-[3-(4-(2-n-Butyloxyethoxy-methyl)phenoxy)-2-hydroxypropylamino]ethoxy]-3-chlor-phenyl]-4,5-dihydro-3(2H)-pyridazinon und
6-[4-[2-[3-(4-(2-Methoxyethoxy-methyl)phenoxy)-2-hydroxypropylamino]ethoxy]-3-methyl-phenyl]-3(2H)-pyridazinon,
deren Tautomere, deren Enantiomere und deren Säureadditionssalze.

5. 2-Hydroxy-n-propylamine der Formel I gemäß Anspruch 1:
6-[4-[2-[3-(4-((2-Isopropoxyethoxy)methyl)phenoxy)-2-hydroxypropyl-amino]ethoxy]phenyl]-4,5-dihydro-3-(2H)-pyridazinon,
6-[4-[2-[3-(4-(2-Isopropoxyethoxy-methyl)phenoxy)-2-hydroxypropylamino]ethoxy]-3-methyl-phenyl]-3(2H)-pyridazinon und
6-[4-[2-[3-(4-(2-n-Butyloxyethoxy-methyl)phenoxy)-2-hydroxypropylamino]ethoxy]-3-chlor-phenyl]-4,5-dihydro-3(2H)-pyridazinon,
deren Tautomere, deren Enantiomere und deren Säureadditionssalze.

6. Physiologisch verträgliche Additionssalze der Verbindungen nach mindestens einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels, das zur Behandlung der myocardialen Ischämie (Angina), zur Behandlung der Herzinsuffizienz, zur Prävention der Herzinsuffizienzprogression nach Myocard-Infarkt und zur Behandlung der Hypertonie geeignet ist.

9. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der 2-Hydroxy-n-propylamine der Formel I gemäß den Ansprüchen 1 bis 6, dadurch gekennzeichnet, daß
eine Verbindung der Formel
$R_1$-O-$CH_2$-CHOH-$CH_2$-Z ,(II)
in der
$R_1$ wie in den Ansprüchen 1 bis 5 definiert ist und
Z eine nukleophile Austrittsgruppe oder
Z zusammen mit dem Wasserstoffatom der benachbarten Hydroxygruppe eine Sauerstoff-Kohlenstoff-Bindung bedeutet, mit einem Amin der Formel
$NH_2$-A-$R_2$ ,(III)
in der
A und $R_2$ wie in den Ansprüchen 1 bis 5 definiert sind, umgesetzt wird und
gewünschtenfalls anschließend eine so erhaltene Verbindung der Formel I in ihre Diastereomeren und in ihre Enantiomeren aufgetrennt wird und/oder
eine so erhaltene Verbindung der Formel I in ihre Säureadditionssalze, insbesondere für die pharmazeutische Anwendung in ihre physiologisch verträglichen Salze mit anorganischen oder organischen Säuren, übergeführt wird.

Europäisches Patentamt

# EUROPÄISCHER RECHERCHENBERICHT

Nummer der Anmeldung: EP 90110052.9

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | US - A - 4 820 819<br>(ROE et al.)<br>* Anspruch 1; Spalte 1, Zeilen 8-25; Beispiel 1 *<br>-- | 1,6,7,9,10 | C 07 D 237/04<br>C 07 D 403/04<br>C 07 D 413/04<br>A 61 K 31/50 |
| A | EP - A2 - 0 259 835<br>(TEIKOKU)<br>* Ansprüche 1,5,7,8,9 *<br>-- | 1-7,9,10 | |
| A | US - A - 3 931 177<br>(COATES et al.)<br>* Spalte 1, Zeile 6 - Spalte 4, Zeile 34 *<br>-- | 1-7,9,10 | |
| A | EP - A1 - 0 310 737<br>(HEUMANN)<br>* Ansprüche 1,13,14 *<br>-- | 1-7,9,10 | |
| A | EP - A2 - 0 272 914<br>(ORTHO)<br>* Ansprüche 1,5 *<br>---- | 1,9,10 | RECHERCHIERTE SACHGEBIETE (Int Cl 5)<br>C 07 D 237/00<br>C 07 D 403/00<br>C 07 D 413/00 |

Der vorliegende Recherchenbericht wurde für alle Patentansprüche erstellt.

| Recherchenort | Abschlußdatum der Recherche | Prüfer |
|---|---|---|
| WIEN | 08-08-1990 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument